(19) Europäisches Patentamt
European Patent Office
Office européen des brevets

(11) EP 4 296 358 A1

(12) **EUROPEAN PATENT APPLICATION**
published in accordance with Art. 153(4) EPC

(43) Date of publication:
**27.12.2023 Bulletin 2023/52**

(21) Application number: **22756320.2**

(22) Date of filing: **21.02.2022**

(51) International Patent Classification (IPC):
***C12N 15/13** (2006.01)* ***A61K 39/395** (2006.01)*
***A61P 37/08** (2006.01)* ***C07K 16/18** (2006.01)*
***C07K 16/44** (2006.01)* ***C07K 17/00** (2006.01)*
***C12N 1/15** (2006.01)* ***C12N 1/19** (2006.01)*
***C12N 5/10** (2006.01)* ***C12N 5/12** (2006.01)*
***G01N 33/50** (2006.01)* ***G01N 33/53** (2006.01)*

(52) Cooperative Patent Classification (CPC):
**A61K 39/395; A61P 37/08; C07K 16/00; C07K 16/18; C07K 16/44; C07K 17/00; C12N 5/10; C12N 5/12; C12N 15/80; C12N 15/81; G01N 33/50; G01N 33/53**

(86) International application number:
**PCT/JP2022/006890**

(87) International publication number:
**WO 2022/177003 (25.08.2022 Gazette 2022/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.02.2021 JP 2021026290**

(71) Applicants:
- **The University of Tokyo**
  **Bunkyo-ku, Tokyo 113-8654 (JP)**
- **Tsuzuki Educational Institute**
  **Fukuoka-shi, Fukuoka 815-8511 (JP)**

(72) Inventors:
- **MURATA, Takahisa**
  **Tokyo 113-8654 (JP)**
- **NAKAMURA, Tatsuro**
  **Tokyo 113-8654 (JP)**
- **NAGATA, Nanae**
  **Tokyo 113-8654 (JP)**
- **ARITAKE, Kosuke**
  **Fukuoka-shi, Fukuoka 815-8511 (JP)**
- **MASUKO, Sakura**
  **Tokyo 113-8654 (JP)**

(74) Representative: **Moodie, Samantha Laura et al**
**Mathys & Squire**
**The Shard**
**32 London Bridge Street**
**London SE1 9SG (GB)**

Remarks:
The complete document including Reference Table(s) and the Sequence Listing(s) can be downloaded from the EPO website

(54) **ANTI-TETRANOR-PGDM MONOCLONAL ANTIBODY AND USE THEREOF**

(57) The present invention relates to a monoclonal antibody or a fragment thereof that specifically binds to tetranor-PGDM.

EP 4 296 358 A1

# Fig. 1

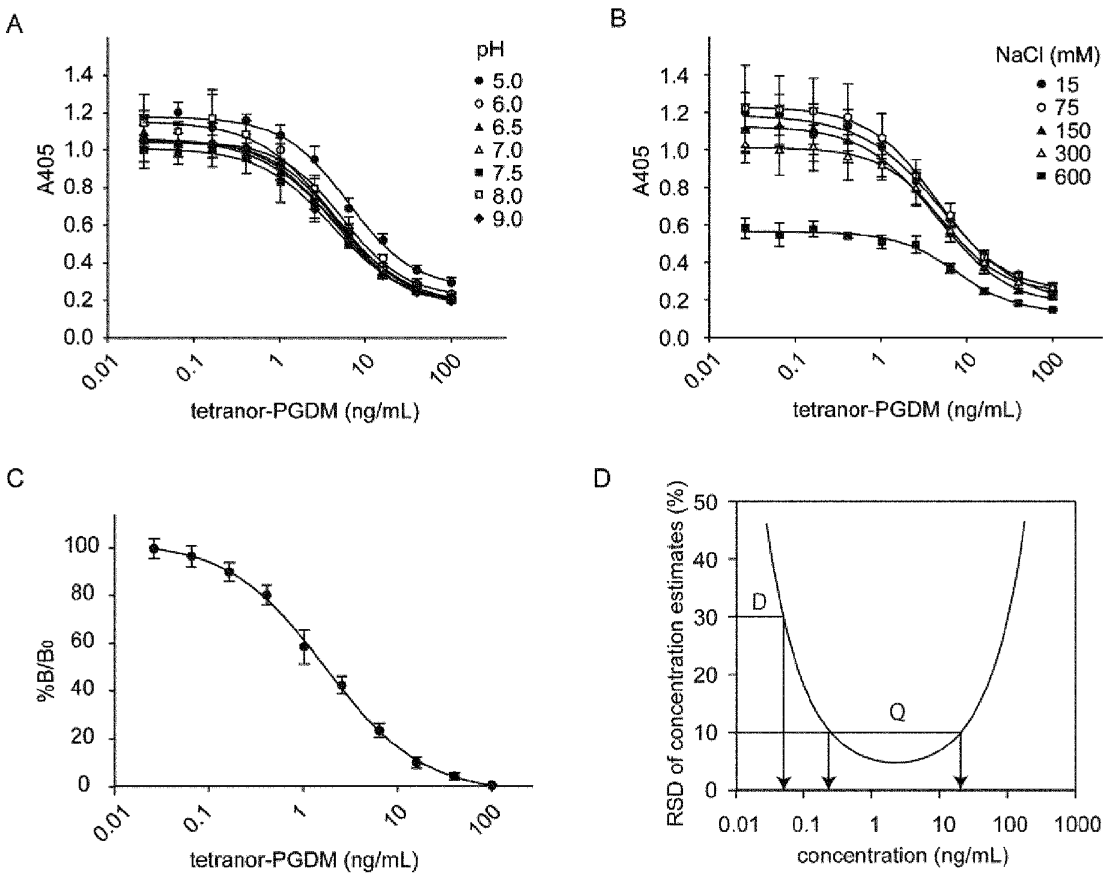
A
pH
5.0
6.0
6.5
7.0
7.5
8.0
9.0
A405
tetranor-PGDM (ng/mL)
B
NaCl (mM)
15
75
150
300
600
A405
tetranor-PGDM (ng/mL)
C
%B/B0
tetranor-PGDM (ng/mL)
D
RSD of concentration estimates (%)
D
Q
concentration (ng/mL)

## Description

### Technical Field

**The** present invention generally relates to a monoclonal antibody or a fragment thereof targeting tetranor-PGDM, a use thereof, and the like.

### Background Art

**A** food allergy is a disease that causes symptoms such as hives, diarrhea, and vomiting due to ingested food and also cause anaphylaxis, which may lead to death. The number of patients with this disease, which is common in children, is rapidly increasing, and it is becoming a more serious problem.

**As** an allergy testing method, measurement of the blood IgE antibody concentration is widely used, but this test does not show whether eating food causes symptoms. Therefore, the only method to make a definitive diagnosis of a food allergy is to perform an "oral antigen load test". However, this requires a great deal of effort and time, and has a risk of causing anaphylaxis. In addition, "oral immunotherapy" may be used as a food allergy treatment, but it is necessary to continue to eat small amounts of the antigen without causing symptoms for several years, which also has a risk of accidents. Thus, the lack of simple and accurate diagnostic methods and symptom evaluation methods has become a bottleneck in the development of methods for diagnosing and treating the increasing number of patients with a food allergy.

The inventors found that tetranor-PGDM (9α-hydroxy-11,15-dioxo-13,14-dihydro-2,3,4,5-tetranor-prostan-1,20-dioic acid), which is a metabolite of lipid prostaglandin $(PG)D_2$, is excreted in urine that is easy to collect even from children, in a manner specific to food allergies and in proportion to the severity of symptoms (Patent Document 1). However, this concentration measurement requires use of a mass spectrometer, which is difficult to operate and expensive.

Citation List

Patent Document

Patent Document 1: WO 2016/021704

Patent Document 2: U.S. Patent Application Publication No. 2012/0021437

### Summary

Technical Problem

Polyclonal antibodies against tetranor-PGDM are known (https://www.caymanchem.com/product/501001/tetranor-pgdm-elisa-kit; U.S. Patent Application Publication No. 2012/0021437 (Patent Document 2)), but polyclonal antibodies are inferior to monoclonal antibodies in terms of specificity, production stability, and measurement reproducibility. In addition to these quality issues, polyclonal antibodies are not applicable for testing human diseases due to lot-to-lot differences in performance.

Therefore, the development of monoclonal antibodies against tetranor-PGDM has been desired, but since tetranor-PGDM has a small molecular weight as an antigen and is difficult for antibodies to recognize, and also exists in vivo, it is difficult to develop monoclonal antibodies against tetranor-PGDM.

Solution to Problem

The inventors succeeded for the first time in preparing a monoclonal antibody specific to tetranor-PGDM by immunizing mice that had no genes encoding two types of $PGD_2$ synthases, a hematopoietic type and a lipocalin type and did not produce $PGD_2$ or its metabolite tetranor-PGDM with a complex of tetranor-PGDM and keyhole limpet hemocyanin (KLH).

Here, in Patent Document 2, a method of producing monoclonal antibodies against tetranor-PGDM by injecting a complex of tetranor-PGDM and keyhole limpet hemocyanin (KLH) into wild-type mice is described, but there is no detailed description of the obtained antibodies. When the inventors performed immunization on wild-type mice in the

same manner, no monoclonal antibody specific to tetranor-PGDM was obtained.

In addition, in Patent Document 2, the cross-reactivity of the antibody against tetranor-PGDM, which is thought to be a polyclonal antibody, is described (paragraph 0347), but the antibody is different from the monoclonal antibody (for example, Tetranor-PGJM 563%; Tetranor-PGDM 100%; Tetranor-PGEM 0.00%) of the present invention, which does not substantially bind to tetranor-PGEM, in that it binds not only to tetranor-PGDM but also to tetranor-PGEM (Tetranor-PGJM 376%; Tetranor-PGDM 100%; Tetranor-PGEM 0.3%). Both antibodies are common in that they bind to tetranor-PGJM, but tetranor-PGJM is hardly produced in vivo and is not excreted in urine. On the other hand, since a large amount of tetranor-PGEM is produced in the body and detected in urine, whether it binds to tetranor-PGEM is a very important difference. Such a difference is thought to be caused by, for example, a difference in CDR.

That is, the present invention encompasses the following inventions.

[1] A monoclonal antibody or a fragment thereof that specifically binds to tetranor-PGDM.

[2] The monoclonal antibody or a fragment thereof according to [1], which does not substantially bind to tetranor-PGEM, tetranor-PGFM, or tetranor-PGAM.

[3] The monoclonal antibody or a fragment thereof according to [1] or [2], which recognizes carbon chains at positions 7 to 11 of tetranor-PGDM.

[4] The monoclonal antibody or a fragment thereof according to any one of [1] to [3], which comprises at least one or more CDRs selected from the group consisting of the following:

(a) CDRH1 consisting of an amino acid sequence as shown in GYT-Xaa1-T-Xaa2-Y-Xaa3 (SEQ ID NO: 1), wherein Xaa1 is F, A or S, Xaa2 is N or R, and Xaa3 is A or W;

(b) CDRH2 consisting of an amino acid sequence as shown in I-Xaa4-P-Xaa5-Xaa6-Xaa7-Xaa8-T (SEQ ID NO: 2), wherein Xaa4 is N or Y, Xaa5 is F or G, Xaa6 is N or D, Xaa7 is D or G, and Xaa8 is G, N or D;

(c) CDRH3 consisting of an amino acid sequence as shown in A-Xaa9-Xaa10-Xaa11-Xaa12-Xaa13-Xaa14-Xaa15-Xaa16-Xaa17-Xaa18-Xaa19-Xaa20 (SEQ ID NO: 3), wherein Xaa9 is R, S or T, Xaa10 is Y or V, Xaa11 is Y or absent, Xaa12 is Y or absent, Xaa13 is Y or absent, Xaa14 is G or S, Xaa15 is S or R, Xaa16 is K or W, Xaa17 is A or F, Xaa18 is M or F, Xaa19 is D or A, and Xaa20 is Y or V;

(d) CDRL1 consisting of an amino acid sequence as shown in Xaa21-GAV-Xaa22-Xaa23-S-Xaa24-Xaa25 (SEQ ID NO: 4), wherein Xaa21 is T or S, Xaa22 is T or R, Xaa23 is T or I, Xaa24 is N, Y or K, and Xaa25 is Y or H;

(e) CDRL2 consisting of an amino acid sequence as shown in Xaa26-T-Xaa27, wherein Xaa26 is G or D, and Xaa27 is N or D; and

(f) CDRL3 consisting of an amino acid sequence as shown in ALW-Xaa28-SNHW-Xaa29 (SEQ ID NO: 5), wherein Xaa28 is Y, F or N, and Xaa29 is V or I;

or comprises at least one or more CDRs consisting of amino acid sequences with deletion, substitution or addition of one or several amino acids in the amino acid sequence of each CDR,

or comprises at least one or more CDRs consisting of amino acid sequences with at least 80% identity to the amino acid sequence of each CDR.

[5] The monoclonal antibody or a fragment thereof according to [4],
wherein, in CDRH1 of (a), when Xaa2 is N, Xaa1 is F, Xaa3 is A; or when Xaa2 is R, Xaa1 is F, A or S.

[6] The monoclonal antibody or a fragment thereof according to [4] or [5],
wherein, in CDRH2 of (b), when Xaa4 is N, Xaa5 is F, Xaa6 is N, Xaa7 is D, and Xaa8 is G; when Xaa4 is Y, Xaa5 is G, Xaa6 is D, Xaa7 is G, and Xaa8 is N or D.

[7] The monoclonal antibody or a fragment thereof according to any one of [4] to [6],
wherein, in CDRH3 of (c), when Xaa10 is Y, Xaa9 is R, Xaa11 is Y, Xaa12 is Y, Xaa13 is Y, Xaa14 is G, Xaa15 is S, Xaa16 is K, Xaa17 is A, Xaa18 is M, Xaa19 is D, and Xaa20 is Y; when Xaa10 is V, Xaa9 is S or T, Xaa11 is

absent, Xaa12 is absent, Xaa13 is absent, Xaa14 is S, Xaa15 is R, Xaa16 is W, Xaa17 is F, Xaa18 is F, Xaa19 is A, and Xaa20 is V.

[8] The monoclonal antibody or a fragment thereof according to any one of [4] to [7],
wherein, in CDRL1 of (d), when Xaa22 and Xaa23 are each T, Xaa21 is T or S, Xaa24 is N, Y or K, and Xaa25 is Y or H; when Xaa22 is R and Xaa23 is I, Xaa21 is T, Xaa24 is N, and Xaa25 is Y.

[9] The monoclonal antibody or a fragment thereof according to any one of [4] to [8],
wherein, in CDRL2 of (e), when Xaa26 is G, Xaa27 is not D.

[10] The monoclonal antibody or a fragment thereof according to any one of [4] to [9],
wherein, in CDRL3 of (f), when Xaa28 is Y, Xaa29 is V or I; when Xaa28 is F, Xaa29 is V; or when Xaa28 is N, Xaa29 is V.

[11] The monoclonal antibody or a fragment thereof according to any one of [1] to [10],
wherein a constant region is derived from a human.

[12] The monoclonal antibody or a fragment thereof according to any one of [1] to [11], which is human chimerized or humanized.

[13] The monoclonal antibody or a fragment thereof according to any one of [1] to [12], which is selected from the group consisting of Fab, F(ab')2, Fab', Fv and single chain antibodies.

[14] A polynucleotide encoding the monoclonal antibody or a fragment thereof according to any one of [1] to [13].

[15] An expression vector including the polynucleotide according to [14].

[16] A host cell transfected with the expression vector according to [15].

[17] The host cell according to [16], which is a eukaryotic cell.

[18] A hybridoma that produces the monoclonal antibody according to any one of [1] to [13].

[19] A diagnostic agent for tetranor-PGDM-related disease, including the monoclonal antibody or a fragment thereof according to any one of [1] to [13].

[20] The diagnostic agent according to [19],
wherein the tetranor-PGDM-related disease is a food allergy, an anaphylactic reaction, muscular dystrophy, or aspirin intolerant asthma.

[21] A kit including the monoclonal antibody or a fragment thereof according to any one of [1] to [13] or the diagnostic agent according to [19] or [20].

[22] The kit according to [21], further including a solid phase carrier,
wherein the monoclonal antibody or a fragment thereof is immobilized on the solid phase carrier.

[23] A method for detecting tetranor-PGDM using the monoclonal antibody or a fragment thereof according to any one of [1] to [13] or the diagnostic agent according to [19] or [20].

[24] A method of assisting in determining whether a subject has a food allergy, including:

a step of detecting a urinary tetranor-PGDM level using the monoclonal antibody or a fragment thereof according to any one of [1] to [13] or using the diagnostic agent according to [19] or [20]; and

a step of assisting in determining that, when the urinary tetranor-PGDM level is higher, food allergy symptoms are severe or have become severe, or a risk of developing a food allergy is higher or has become higher.

[25] The method according to [24], further including a step of measuring a urinary tetranor-PGEM level.

Advantageous Effects of Invention

Using the anti-tetranor-PGDM monoclonal antibody of the present invention, tetranor-PGDM, which is conventionally measured by mass spectrometry, can be simply detected through enzyme immunoassay or the like. Such an enzyme immunoassay method is expected to be useful for quantification of tetranor-PGDM in body fluids, evaluation of animal disease models, diagnosis indicators, and treatment monitoring of food allergies and other diseases.

**Brief Description of Drawings**

Fig. 1 shows the results obtained by examining influences of a pH value (A) and an ionic strength (B) on performance of enzyme immunoassay using an anti-tetranor-PGDM monoclonal antibody; and shows a competition curve of tetranor-PGDM under optimized conditions (C) and the results obtained by examining the limit of detection of tetranor-PGDM (D).

Fig. 2 shows structures of tetranor-PGDM, tetranor-PGEM, tetranor-PGFM, and tetranor-PGAM.

Fig. 3 shows the results measured by gradually diluting tetranor-PGDM recovered from artificial urine before (A) and after (B) solid phase extraction.

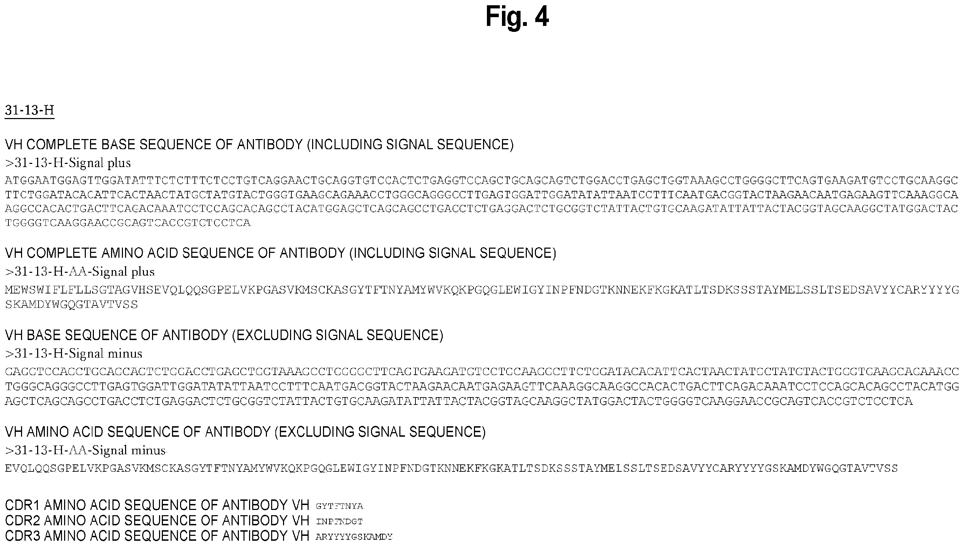

Fig. 4 shows the sequence of the 31-13 antibody H chain.

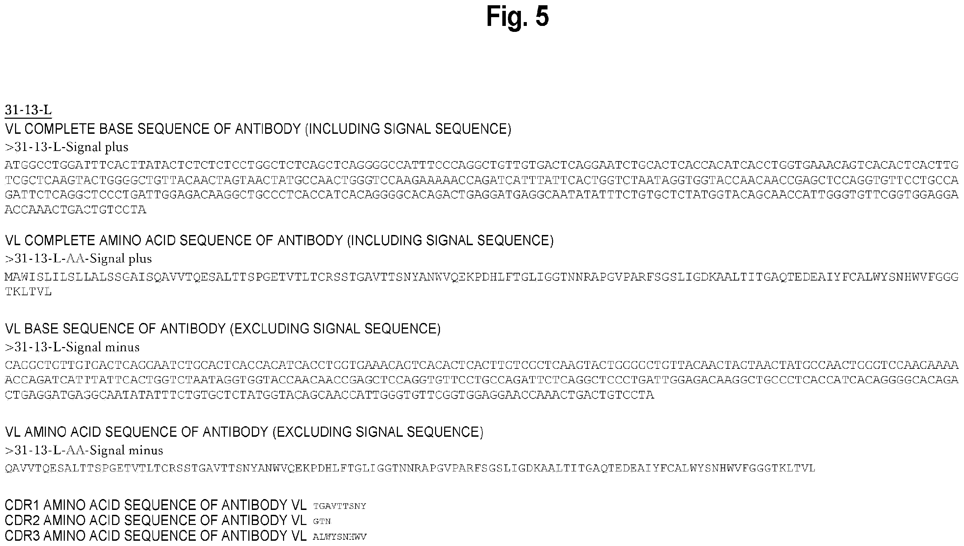

Fig. 5 shows the sequence of the 31-13 antibody L chain.

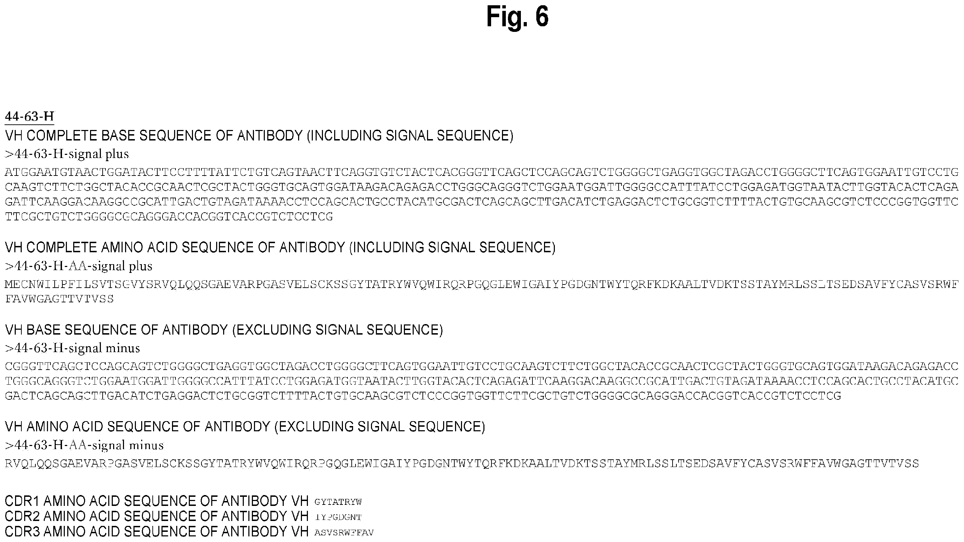

Fig. 6 shows the sequence of the 44-63 antibody H chain.

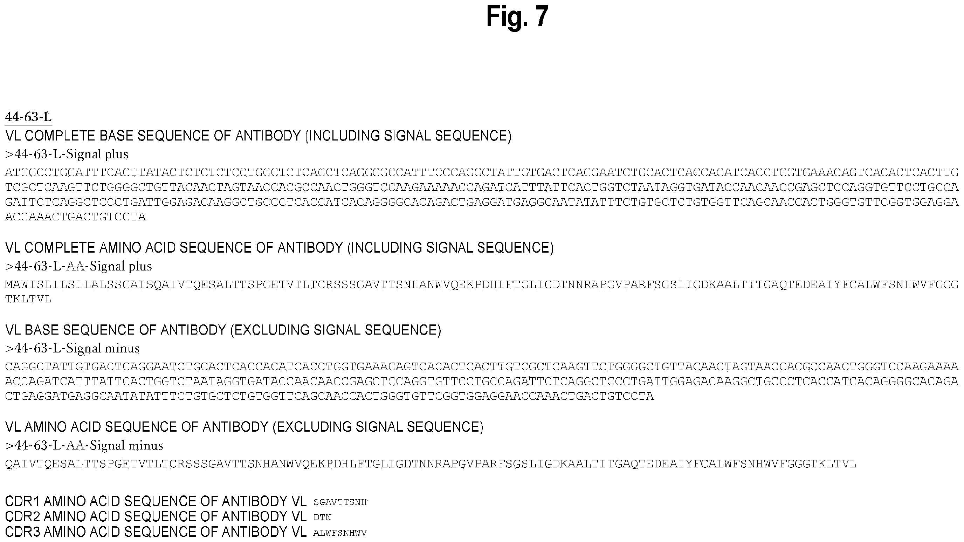

Fig. 7 shows the sequence of the 44-63 antibody L chain.

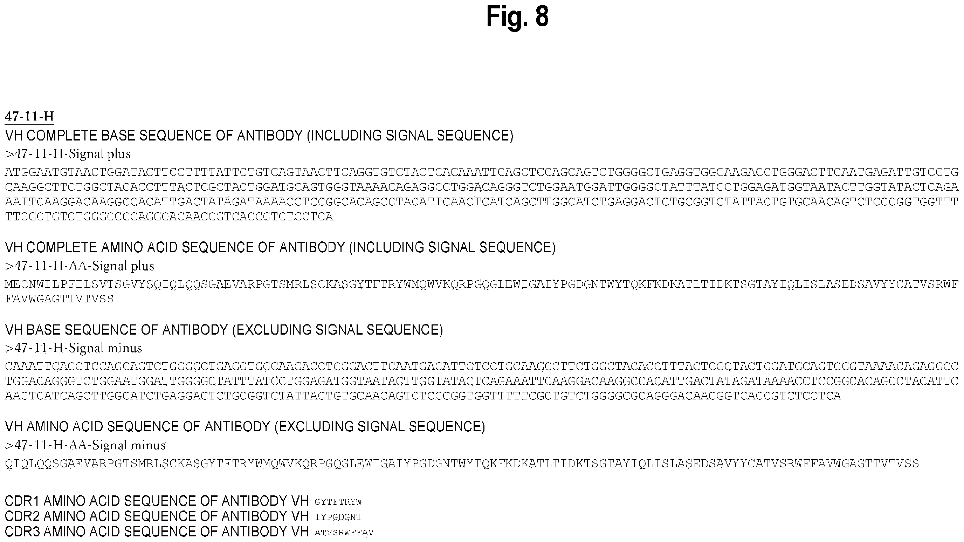

Fig. 8 shows the sequence of the 47-11 antibody H chain.

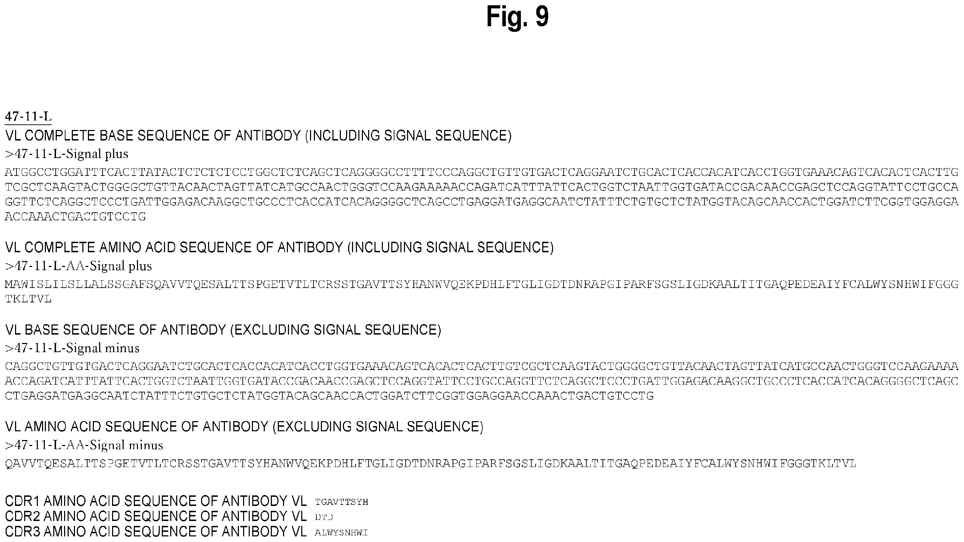

Fig. 9 shows the sequence of the 47-11 antibody L chain.

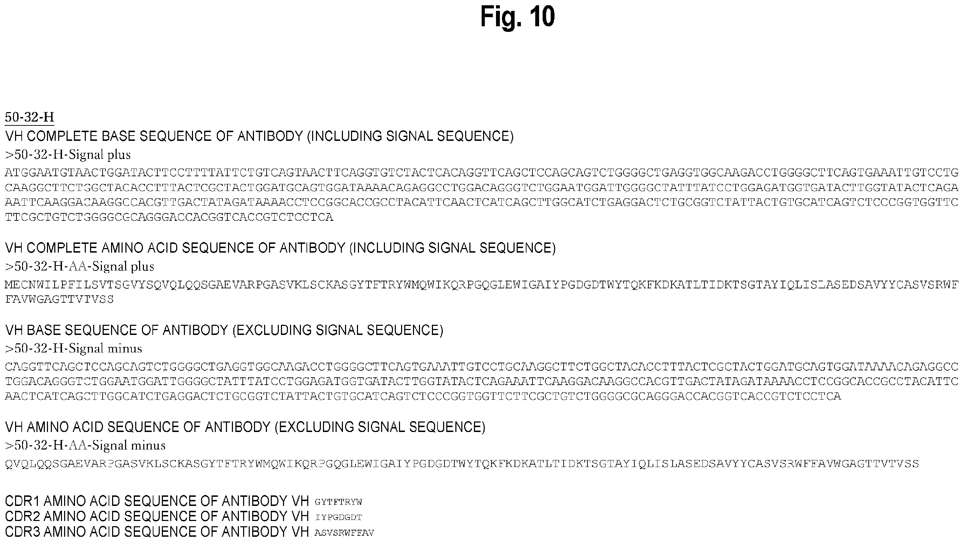

Fig. 10 shows the sequence of the 50-32 antibody H chain.

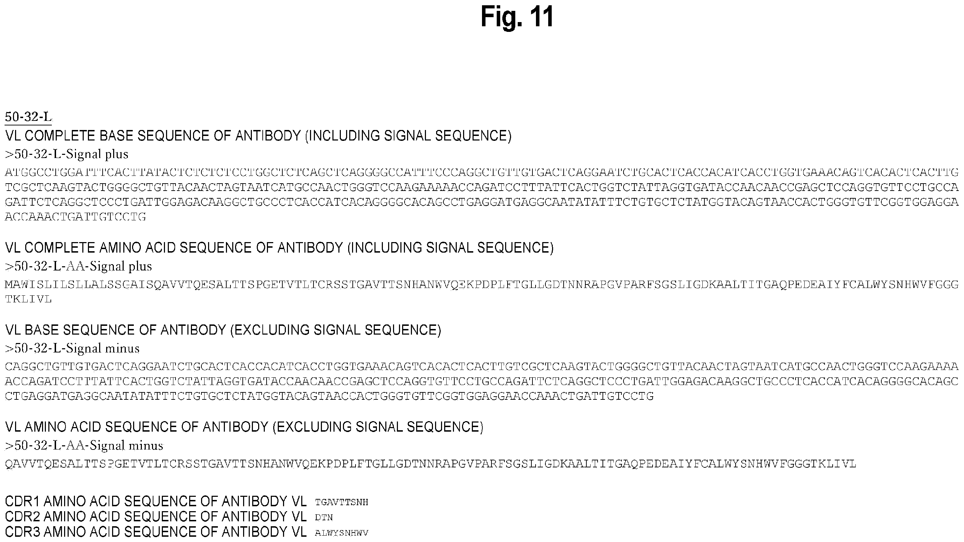

Fig. 11 shows the sequence of the 50-32 antibody L chain.

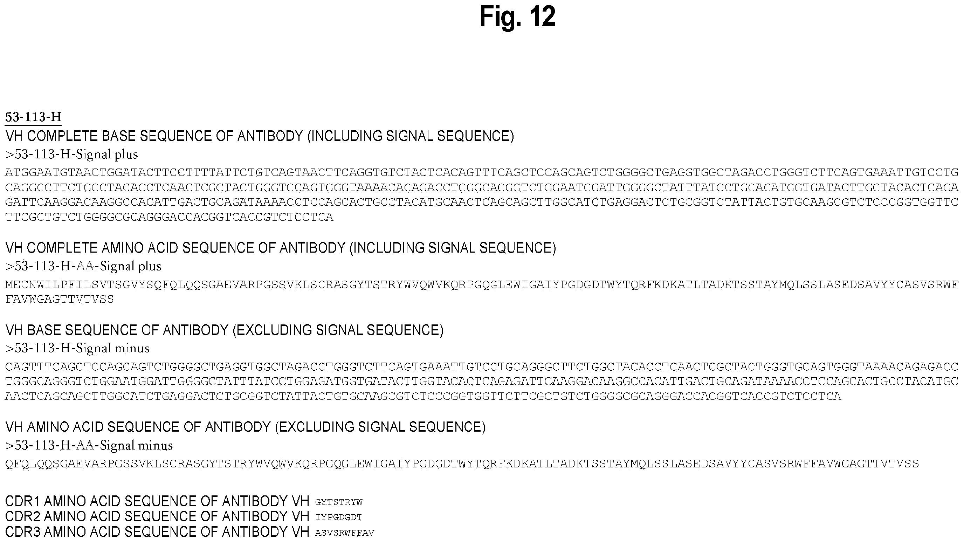

Fig. 12 shows the sequence of the 53-113 antibody H chain.

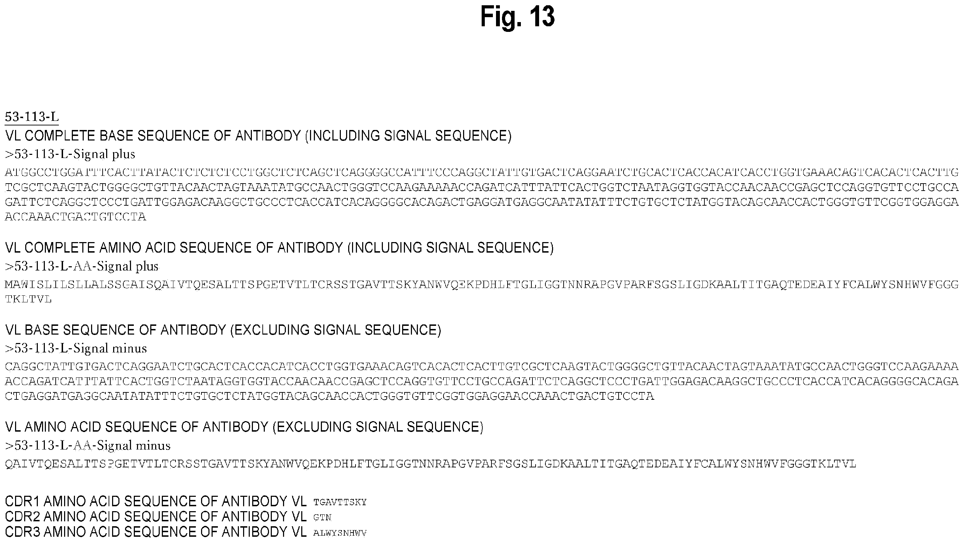

Fig. 13 shows the sequence of the 53-113 antibody L chain.

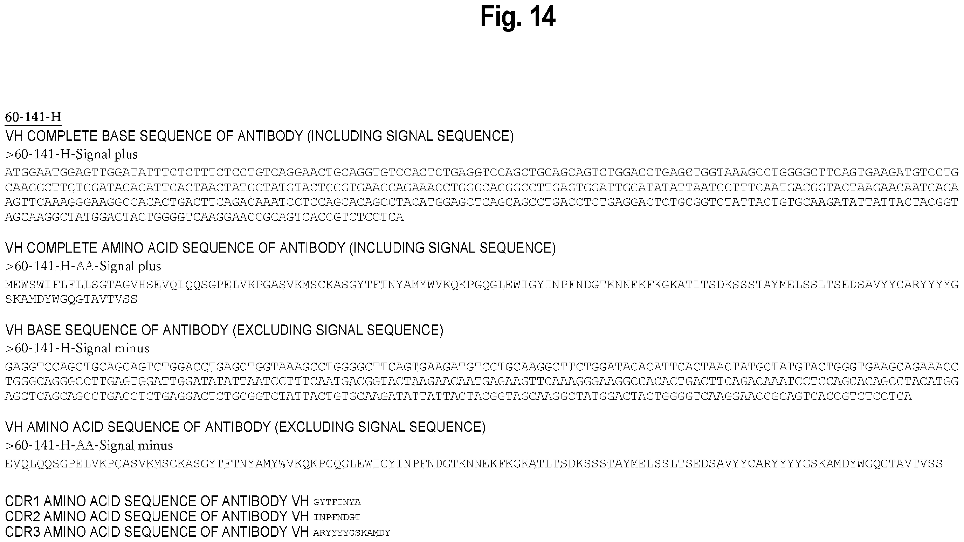

Fig. 14 shows the sequence of 60-141 antibody H chain.

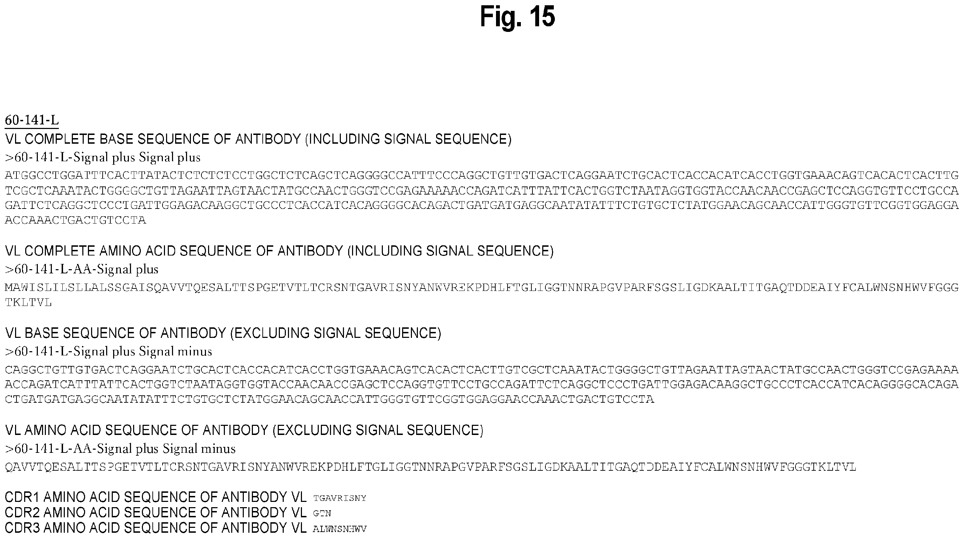

Fig. 15 shows the sequence of the 60-141 antibody L chain.

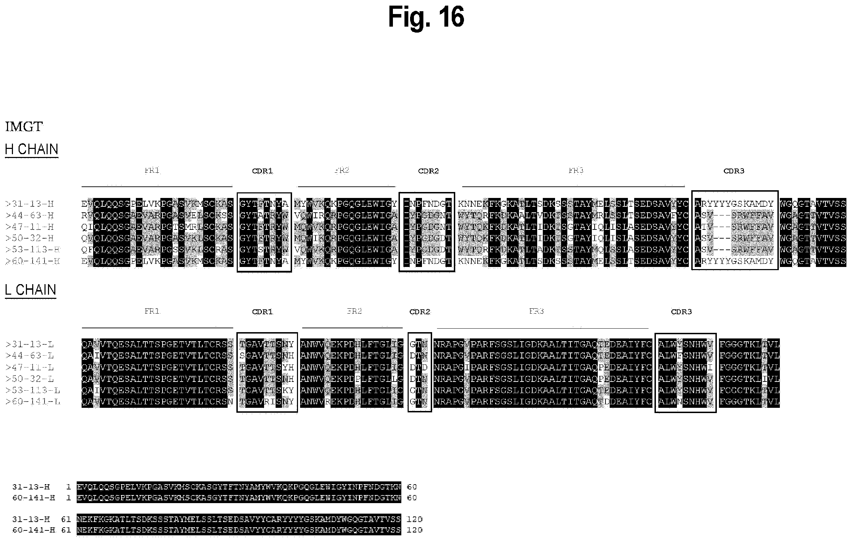

Fig. 16 shows alignments of amino acid sequences of each antibody.

**Description of Embodiments**

(Anti-tetranor-PGDM monoclonal antibody)

In a first aspect, a monoclonal antibody or a fragment thereof that specifically binds to tetranor-PGDM is provided. As used herein, this antibody hereinafter will be simply referred to as an anti-tetranor-PGDM monoclonal antibody. As used herein, "specifically binds to tetranor-PGDM" means that an antibody does not substantially bind to tetranor-PGEM, tetranor-PGFM, or tetranor-PGAM, and specifically binds to tetranor-PGDM or tetranor-PGJM. A monoclonal antibody that does not bind to tetranor-PGEM is preferable because it is excreted particularly abundantly in body fluids including urine. Here, since tetranor-PGJM is hardly produced in vivo and is not excreted in urine, it can be said that the anti-tetranor-PGDM monoclonal antibody is substantially a tetranor-PGDM-specific antibody.

Anti-tetranor-PGDM monoclonal antibodies can be produced using hybridomas obtained by fusing antibody-producing cells with myelomas. The produced antibodies may be further subjected to an additional step such as a purifying step before recovery, and for example, in order to increase the purity, it is preferable to perform salting-out, filter filtration, clarification using centrifugation, recovery by affinity, intermediate purification by ion exchange, and final purification by gel filtration.

Examples of anti-tetranor-PGDM monoclonal antibodies obtained in this manner include antibodies including at least one or more CDRs and preferably all six CDRs selected from the group consisting of the following complementarity-determining regions (CDRs):

(a) CDRH1 consisting of an amino acid sequence as shown in GYT-Xaa1-T-Xaa2-Y-Xaa3 (SEQ ID NO: 1), wherein Xaa1 is F, A or S, Xaa2 is N or R, and Xaa3 is A or W;

(b) CDRH2 consisting of an amino acid sequence as shown in I-Xaa4-P-Xaa5-Xaa6-Xaa7-Xaa8-T (SEQ ID NO: 2), wherein Xaa4 is N or Y, Xaa5 is F or G, Xaa6 is N or D, Xaa7 is D or G, and Xaa8 is G, N or D;

(c) CDRH3 consisting of an amino acid sequence as shown in A-Xaa9-Xaa10-Xaa11-Xaa12-Xaa13-Xaa14-Xaa15-Xaa16-Xaa17-Xaa18-Xaa19-Xaa20 (SEQ ID NO: 3), wherein Xaa9 is R, S or T, Xaa10 is Y or V, Xaa11 is Y or absent, Xaa12 is Y or absent, Xaa13 is Y or absent, Xaa14 is G or S, Xaa15 is S or R, Xaa16 is K or W, Xaa17 is A or F, Xaa18 is M or F, Xaa19 is D or A, and Xaa20 is Y or V;

(d) CDRL1 consisting of an amino acid sequence as shown in Xaa21-GAV-Xaa22-Xaa23-S-Xaa24-Xaa25 (SEQ ID NO: 4), wherein Xaa21 is T or S, Xaa22 is T or R, Xaa23 is T or I, Xaa24 is N, Y or K, and Xaa25 is Y or H;

(e) CDRL2 consisting of an amino acid sequence as shown in Xaa26-T-Xaa27, wherein Xaa26 is G or D, and Xaa27 is N or D; and

(f) CDRL3 consisting of an amino acid sequence as shown in ALW-Xaa28-SNHW-Xaa29 (SEQ ID NO: 5), wherein Xaa28 is Y, F or N, and Xaa29 is V or I.

Antibodies generally have a structure in which two heavy chains and two light chains are covalently bound by disulfide bonds, and the heavy chains are divided into a heavy chain variable region and a heavy chain constant region, and the light chains are divided into a light chain variable region and a light chain constant region. Each variable region comprises three CDRs. Here, CDRH1, CDRH2, and CDRH3 represent the complementarity-determining regions of the heavy chain in order from the amino terminal side of the heavy chain amino acid sequence, and CDRL1, CDRL2, and CDRL3 represent the complementarity-determining regions of the light chain in order from the amino terminal side of the light chain amino acid sequence.

Therefore, the anti-tetranor-PGDM monoclonal antibody or a fragment thereof may be an antibody or a fragment thereof which preferably comprises a heavy chain variable region including:

(a) CDRH1 consisting of an amino acid sequence as shown in GYT-Xaa1-T-Xaa2-Y-Xaa3 (SEQ ID NO: 1), wherein Xaa1 is F, A or S, Xaa2 is N or R, and Xaa3 is A or W;

(b) CDRH2 consisting of an amino acid sequence as shown in I-Xaa4-P-Xaa5-Xaa6-Xaa7-Xaa8-T (SEQ ID NO: 2), wherein Xaa4 is N or Y, Xaa5 is F or G, Xaa6 is N or D, Xaa7 is D or G, and Xaa8 is G, N or D; and

(c) CDRH3 consisting of an amino acid sequence as shown in A-Xaa9-Xaa10-Xaa11-Xaa12-Xaa13-Xaa14-Xaa15-Xaa16-Xaa17-Xaa18-Xaa19-Xaa20 (SEQ ID NO: 3), wherein Xaa9 is R, S or T, Xaa10 is Y or V, Xaa11 is Y or absent, Xaa12 is Y or absent, Xaa13 is Y or absent, Xaa14 is G or S, Xaa15 is S or R, Xaa16 is K or W, Xaa17 is A or F, Xaa18 is M or F, Xaa19 is D or A, and Xaa20 is Y or V; and/or a light chain variable region including:

(d) CDRL1 consisting of an amino acid sequence as shown in Xaa21-GAV-Xaa22-Xaa23-S-Xaa24-Xaa25 (SEQ ID NO: 4), wherein Xaa21 is T or S, Xaa22 is T or R, Xaa23 is T or I, Xaa24 is N, Y or K, and Xaa25 is Y or H;

(e) CDRL2 consisting of an amino acid sequence as shown in Xaa26-T-Xaa27, wherein Xaa26 is G or D, and Xaa27 is N or D; and

(f) CDRL3 consisting of an amino acid sequence as shown in ALW-Xaa28-SNHW-Xaa29 (SEQ ID NO: 5), wherein Xaa28 is Y, F or N, and Xaa29 is V or I.

The amino acid sequence of each CDR may have deletion, substitution or addition of one or several amino acids as long as the anti-tetranor-PGDM monoclonal antibody or a fragment thereof specifically binds to tetranor-PGDM. As used herein, although it varies depending on the length of the sequence, "several" is 2 to 10, preferably 2 to 5, and more preferably 2 or 3.

The amino acid after substitution is desirably an amino acid that preserves properties of the original amino acid side chains as classified below. However, amino acid substitutions are not limited to conservative substitutions.

hydrophobic amino acids (A, I, L, M, F, P, W, Y, V;

hydrophilic amino acids (R, D, N, C, E, Q, G, H, K, S, T);

amino acids with aliphatic side chains (G, A, V, L, I, P);

amino acids with hydroxyl group-containing side chains (S, T, Y);

amino acids with sulfur atom-containing side chains (C, M);

amino acids with carboxylic acid- and amide-containing side chains (D, N, E, Q);

amino acids with base-containing side chains (R, K, H);

amino acids with aromatic-containing side chains (H, F, Y, W)

In one embodiment, amino acid substitutions may occur at amino acids present at corresponding positions in a natural human antibody.

In one embodiment, amino acid substitutions may occur outside of the complementarity-determining region (CDR).

In one embodiment, amino acid substitutions may occur in the heavy chain variable region or the light chain variable region.

Alternatively, the amino acid sequence of each CDR may be consisting of amino acid sequences having a homology of 80% or more, and preferably identity of 80% or more with respect to the sequence shown in each SEQ ID NO as long as it specifically binds to tetranor-PGDM. The homology or identity of the sequence is preferably 81%, 82%, 83%, 84%, 85%, 86%, 87%, 88%, 89%, 90%, 91%, 92%, 93%, 94%, 95%, 96%, 97%, 98%, or 99% or more, and more preferably 99% or more.

In one embodiment, the anti-tetranor-PGDM monoclonal antibody may be an antibody or a fragment thereof that binds to an epitope to which an antibody or a fragment thereof binds, which comprises a variable region including at least one or more CDRs selected from the group consisting of the following:

(a) CDRH1 consisting of an amino acid sequence as shown in GYT-Xaa1-T-Xaa2-Y-Xaa3 (SEQ ID NO: 1), wherein Xaa1 is F, A or S, Xaa2 is N or R, and Xaa3 is A or W;

(b) CDRH2 consisting of an amino acid sequence as shown in I-Xaa4-P-Xaa5-Xaa6-Xaa7-Xaa8-T (SEQ ID NO: 2), wherein Xaa4 is N or Y, Xaa5 is F or G, Xaa6 is N or D, Xaa7 is D or G, and Xaa8 is G, N or D;

(c) CDRH3 consisting of an amino acid sequence as shown in A-Xaa9-Xaa10-Xaa11-Xaa12-Xaa13-Xaa14-Xaa15-Xaa16-Xaa17-Xaa18-Xaa19-Xaa20 (SEQ ID NO: 3), wherein Xaa9 is R, S or T, Xaa10 is Y or V, Xaa11 is Y or absent, Xaa12 is Y or absent, Xaa13 is Y or absent, Xaa14 is G or S, Xaa15 is S or R, Xaa16 is K or W, Xaa17 is A or F, Xaa18 is M or F, Xaa19 is D or A, and Xaa20 is Y or V;

(d) CDRL1 consisting of an amino acid sequence as shown in Xaa21-GAV-Xaa22-Xaa23-S-Xaa24-Xaa25 (SEQ ID NO: 4), wherein Xaa21 is T or S, Xaa22 is T or R, Xaa23 is T or I, Xaa24 is N, Y or K, and Xaa25 is Y or H;

(e) CDRL2 consisting of an amino acid sequence as shown in Xaa26-T-Xaa27, wherein Xaa26 is G or D, and Xaa27 is N or D; and

(f) CDRL3 consisting of an amino acid sequence as shown in ALW-Xaa28-SNHW-Xaa29 (SEQ ID NO: 5), wherein Xaa28 is Y, F or N, and Xaa29 is V or I,

or comprises at least one or more variable regions including CDRs consisting of amino acid sequences with deletion, substitution or addition of one or several amino acids in the amino acid sequence of each CDR,

or comprises at least one or more variable regions including CDRs consisting of amino acid sequences with at least 80% identity to the amino acid sequence of each CDR.

As used herein, "epitope" means a partial structure of tetranor-PGDM to which an anti-tetranor-PGDM monoclonal antibody or a fragment thereof binds. As such a partial structure, carbon chains at positions 7 to 11 of tetranor-PGDM represented by the following chemical formula:

[C1]

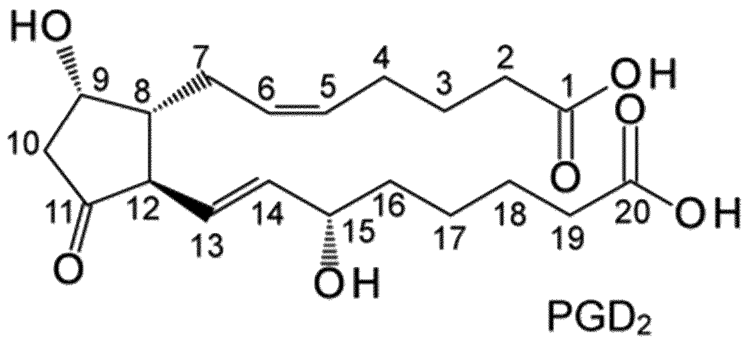

$PGD_2$

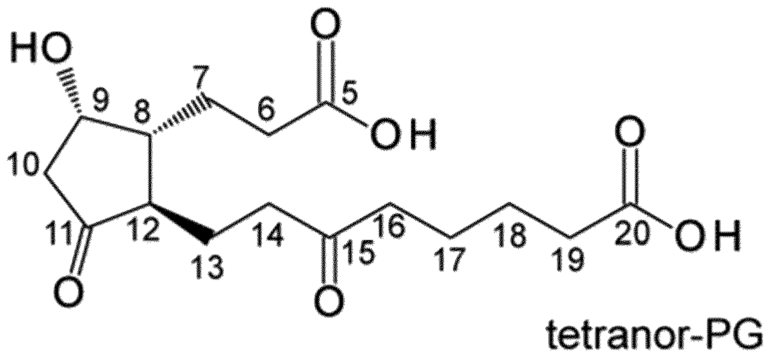

tetranor-PGDM

,

particularly the 11-keto group, can be considered. The 7th to 11th positions are numbered based on the carbon number of $PGD_2$, and the 1st to 4th positions are missing numbers.

In one embodiment, the anti-tetranor-PGDM monoclonal antibody may be an antibody or a fragment thereof which comprises a heavy chain variable region including the following:

(a) CDRH1 consisting of an amino acid sequence as shown in GYT-Xaa1-T-Xaa2-Y-Xaa3 (SEQ ID NO: 1), wherein Xaa1 is F, A or S, Xaa2 is N or R, and Xaa3 is A or W;

(b) CDRH2 consisting of an amino acid sequence as shown in I-Xaa4-P-Xaa5-Xaa6-Xaa7-Xaa8-T (SEQ ID NO: 2), wherein Xaa4 is N or Y, Xaa5 is F or G, Xaa6 is N or D, Xaa7 is D or G, and Xaa8 is G, N or D; and

(c) CDRH3 consisting of an amino acid sequence as shown in A-Xaa9-Xaa10-Xaa11-Xaa12-Xaa13-Xaa14-Xaa15-Xaa16-Xaa17-Xaa18-Xaa19-Xaa20 (SEQ ID NO: 3), wherein Xaa9 is R, S or T, Xaa10 is Y or V, Xaa11 is Y or absent, Xaa12 is Y or absent, Xaa13 is Y or absent, Xaa14 is G or S, Xaa15 is S or R, Xaa16 is K or W, Xaa17 is A or F, Xaa18 is M or F, Xaa19 is D or A, and Xaa20 is Y or V,

or comprises a heavy chain variable region including CDRH1, CDRH2 and CDRH3 consisting of amino acid sequences with deletion, substitution or addition of one or several amino acids in the amino acid sequence of each CDR,

or comprises a heavy chain variable region including CDRH1, CDRH2 and CDRH3 consisting of amino acid sequences with at least 80% identity to the amino acid sequence of each CDR.

In one embodiment, the heavy chain variable region in the anti-tetranor-PGDM monoclonal antibody or a fragment thereof may comprise at least 109 consecutive amino acids of the amino acid sequence shown in any of SEQ ID NOs: 9, 22, 35, 48, 61, and 74.

As used herein, "at least 109 consecutive amino acids of the amino acid sequence shown in any of SEQ ID NOs: 9, 22, 35, 48, 61, and 74" indicates 109, 110, 112 or more consecutive amino acids in the amino acid sequence shown in any of SEQ ID NOs: 9, 22, 35, 48, 61, and 74. Such consecutive amino acids preferably comprise amino acid sequences of the above CDRH1, CDRH2 and CDRH3.

In one embodiment, the heavy chain variable region in the anti-tetranor-PGDM monoclonal antibody or a fragment thereof may comprise amino acid sequences with deletion, substitution or addition of one or several amino acids in the amino acid sequence shown in any of SEQ ID NOs: 9, 22, 35, 48, 61, and 74 and the amino acid sequence shown in any of SEQ ID NOs: 9, 22, 35, 48, 61, and 74, or amino acid sequence or amino acid sequences with at least 80% identity to the amino acid sequence shown in any of SEQ ID NOs: 9, 22, 35, 48, 61, and 74.

In one embodiment, the anti-tetranor-PGDM monoclonal antibody may be an antibody or a fragment thereof which comprises a light chain variable region including the following:

(d) CDRL1 consisting of an amino acid sequence as shown in Xaa21-GAV-Xaa22-Xaa23-S-Xaa24-Xaa25 (SEQ ID NO: 4), wherein Xaa21 is T or S, Xaa22 is T or R, Xaa23 is T or I, Xaa24 is N, Y or K, and Xaa25 is Y or H;

(e) CDRL2 consisting of an amino acid sequence as shown in Xaa26-T-Xaa27, wherein Xaa26 is G or D, and Xaa27 is N or D; and

(f) CDRL3 consisting of an amino acid sequence as shown in ALW-Xaa28-SNHW-Xaa29 (SEQ ID NO: 5), wherein Xaa28 is Y, F or N, and Xaa29 is V or I;

or comprises a light chain variable region including CDRL1, CDRL2 and CDRL3 consisting of amino acid sequences with deletion, substitution or addition of one or several amino acids in the amino acid sequence of each CDR,

or comprises a light chain variable region including CDRL1, CDRL2 and CDRL3 consisting of amino acid sequences with at least 80% identity to the amino acid sequence of each CDR.

In one embodiment, the anti-tetranor-PGDM monoclonal antibody may be an antibody or a fragment thereof which comprises a variable region including the following:

(a) CDRH1 consisting of an amino acid sequence as shown in GYT-Xaa1-T-Xaa2-Y-Xaa3 (SEQ ID NO: 1), wherein Xaa1 is F, A or S, Xaa2 is N or R, and Xaa3 is A or W;

(b) CDRH2 consisting of an amino acid sequence as shown in I-Xaa4-P-Xaa5-Xaa6-Xaa7-Xaa8-T (SEQ ID NO: 2), wherein Xaa4 is N or Y, Xaa5 is F or G, Xaa6 is N or D, Xaa7 is D or G, and Xaa8 is G, N or D;

(c) CDRH3 consisting of an amino acid sequence as shown in A-Xaa9-Xaa10-Xaa11-Xaa12-Xaa13-Xaa14-Xaa15-Xaa16-Xaa17-Xaa18-Xaa19-Xaa20 (SEQ ID NO: 3), wherein Xaa9 is R, S or T, Xaa10 is Y or V, Xaa11 is Y or absent, Xaa12 is Y or absent, Xaa13 is Y or absent, Xaa14 is G or S, Xaa15 is S or R, Xaa16 is K or W, Xaa17 is A or F, Xaa18 is M or F, Xaa19 is D or A, and Xaa20 is Y or V;

(d) CDRL1 consisting of an amino acid sequence as shown in Xaa21-GAV-Xaa22-Xaa23-S-Xaa24-Xaa25 (SEQ ID NO: 4), wherein Xaa21 is T or S, Xaa22 is T or R, Xaa23 is T or I, Xaa24 is N, Y or K, and Xaa25 is Y or H;

(e) CDRL2 consisting of an amino acid sequence as shown in Xaa26-T-Xaa27, wherein Xaa26 is G or D, and Xaa27 is N or D; and

(f) CDRL3 consisting of an amino acid sequence as shown in ALW-Xaa28-SNHW-Xaa29 (SEQ ID NO: 5), wherein Xaa28 is Y, F or N, and Xaa29 is V or I;

or comprises a variable region including CDRH1, CDRH2 and CDRH3 and CDRL1, CDRL2 and CDRL3 consisting of amino acid sequences with deletion, substitution or addition of one or several amino acids in the amino acid sequence of each CDR,

or comprises a variable region including CDRH1, CDRH2 and CDRH3 and CDRL1, CDRL2 and CDRL3 consisting of amino acid sequences with at least 80% identity to the amino acid sequence of each CDR.

In one embodiment, when Xaa2 in CDRH1 is N, Xaa1 may be F, and Xaa3 may be A; or when Xaa2 is R, Xaa1 may be F, A or S.

Xaa1 is preferably F. Xaa2 is preferably N. Xaa3 is preferably A. CDRH1 in which Xaa1 is F, Xaa2 is N, and Xaa3 is A is preferable.

In one embodiment, when Xaa4 in CDRH2 is N, Xaa5-Xaa6-Xaa7- Xaa8 may be FNDG; or when Xaa4 is Y, Xaa5-Xaa6-Xaa7 may be GDG, and Xaa8 may be N or D.

Xaa4 is preferably N. Xaa5-Xaa6-Xaa7- Xaa8 is preferably FNDG. CDRH2 in which Xaa4 is N, and Xaa5-Xaa6-Xaa7- Xaa8 is FNDG is preferable.

In one embodiment, when Xaa10 in CDRH3 is Y, Xaa9 may be R, Xaa11 may be Y, Xaa12 may be Y, Xaa13 may be Y, Xaa14 may be G, Xaa15 may be S, Xaa16 may be K, Xaa17 may be A, Xaa18 may be M, Xaa19 may be D, and Xaa20 may be Y; when Xaa10 is V, Xaa9 may be S or T, Xaa11 may be absent, Xaa12 may be absent, Xaa13 may be absent, Xaa14 may be S, Xaa15 may be R, Xaa16 may be W, Xaa17 may be F, Xaa18 may be F, and Xaa19 may be A, Xaa20 may be V.

Xaa9 is preferably R. Xaa10 is preferably Y. It is preferable that Xaa11 be present and particularly be Y. It is preferable that Xaa12 be present and particularly be Y. It is preferable that Xaa13 be present and particularly be Y. Xaa14 is preferably G. Xaa15 is preferably S. Xaa16 is preferably K. Xaa17 is preferably A. Xaa18 is preferably M. Xaa19 is preferably D. Xaa20 is preferably Y. CDRH3 in which Xaa9 is R, Xaa10 is Y, Xaa11 is Y, Xaa12 is Y, Xaa13 is Y, Xaa14 is G, Xaa15 is S, Xaa16 is K, Xaa17 is A, Xaa18 is M, Xaa19 is D, and Xaa20 is Y is preferable.

In one embodiment, when Xaa22 and Xaa23 in CDRL1 are each T, Xaa21 may be T or S, Xaa24 may be N, Y or K, and Xaa25 may be Y or H; or when Xaa22 is R and Xaa23 is I, Xaa21 may be T, and Xaa24 may be N, and Xaa25 may be Y.

Xaa21 is preferably T. Xaa22 is preferably T or R. Xaa23 is preferably T or I. Xaa24 is preferably N. CDRL1 in which Xaa21 is T, Xaa22 is T or R, Xaa23 is T or I, Xaa24 is N, and Xaa25 is Y is preferable. CDRL1 in which Xaa21 is T, Xaa22 is R, Xaa23 is I, Xaa24 is N, and Xaa25 is Y is more preferable.

In one embodiment, when Xaa26 in CDRL2 is G, Xaa27 may not be D.

Xaa26 is preferably G. Xaa27 is preferably N. CDRL2 in which Xaa26 is G, and Xaa27 is N is preferable.

In one embodiment, when Xaa28 in CDRL3 is Y, Xaa29 may be V or I; when Xaa28 is F, Xaa29 may be V; or when Xaa28 is N, Xaa29 may be V. CDRL3 in which Xaa28 is Y or N, and Xaa29 is V is preferable.

In one embodiment, the light chain variable region in the anti-tetranor-PGDM monoclonal antibody or a fragment thereof may comprise at least 99 consecutive amino acids of the amino acid sequence shown in any of SEQ ID NOs: 95 to 99.

As used herein, "at least 99 consecutive amino acids of the amino acid sequence shown in any of SEQ ID NOs: 16, 29, 42, 55, 68, and 81" indicates 99, 100, 101, 102 or more consecutive amino acids in the amino acid sequence shown in any of SEQ ID NOs: 16, 29, 42, 55, 68, and 81. Such consecutive amino acids preferably comprise the above CDRL1, CDRL2 and CDRL3.

In one embodiment, the light chain variable region in the anti-tetranor-PGDM monoclonal antibody or a fragment thereof may comprise amino acid sequence with deletion, substitution or addition of one or several amino acids in the amino acid sequence shown in any of SEQ ID NOs: 16, 29, 42, 55, 68, and 81 and the amino acid sequence shown in any of SEQ ID NOs: 16, 29, 42, 55, 68, and 81 or amino acid sequences with at least 80% identity to the amino acid sequence shown in any of SEQ ID NOs: 16, 29, 42, 55, 68, and 81.

As long as the anti-tetranor-PGDM monoclonal antibody comprises the above CDRs, other structures, for example, the structures of variable regions and constant regions other than CDRs, are not particularly limited, and examples of amino acid sequences encoding the heavy chain variable region containing the above CDRH1, CDRH2 and CDRH3 and the light chain variable region containing the CDRL1, CDRL2 and CDRL3 comprise amino acid sequences of SEQ ID NOs: 9, 22, 35, 48, 61, and 74 and amino acid sequences of SEQ ID NOs: 16, 29, 42, 55, 68, and 81.

In a preferable embodiment, the anti-tetranor-PGDM monoclonal antibody or a fragment thereof may comprise a heavy chain variable region having an amino acid sequence of SEQ ID NOs: 9, 22, 35, 48, 61, and 74 and a light chain variable region having an amino acid sequence of any of SEQ ID NOs: 16, 29, 42, 55, 68, and 81.

In a preferable embodiment, the humanized anti-tetranor-PGDM monoclonal antibody or a fragment thereof may comprise a heavy chain variable region having an amino acid sequence of any of SEQ ID NOs: 9, 22, 35, 48, 61, and 74 and a light chain variable region having an amino acid sequence of any of SEQ ID NOs: 16, 29, 42, 55, 68, and 81. (examples of combinations: SEQ ID NO: 9 and SEQ ID NO: 16; SEQ ID NO: 22 and SEQ ID NO: 29; SEQ ID NO: 35

and SEQ ID NO: 42; SEQ ID NO: 48 and SEQ ID NO: 55; SEQ ID NO: 74 and SEQ ID NO: 81)

As used herein, "antibody" refers to one that occurs naturally or a wholly or partially synthetically produced artificial immunoglobulin or a fragment thereof. Antibodies may be of any isotype of IgG, IgM, IgA, IgE, and IgD. Nine types of classes (isotypes) of IgG1, IgG2, IgG3, IgG4, IgA1, IgA2, IgD, IgE, and IgM are known as human immunoglobulins.

Without limitation on the origin of antibodies, any species of mammals, for example, humans, and non-human animals such as mice, rats, guinea pigs, hamsters, rabbits, goats, sheep, and camels, may be used. Not only human antibodies but also recombinant antibodies such as humanized antibodies and chimeric antibodies can be used. As used herein, "humanized antibody" is one in which the CDRs of a human antibody are replaced with CDRs derived from a non-human animal antibody. In addition to the CDRs, some framework amino acid residues may be grafted into the human antibody. When CDRs derived from non-human animal antibodies are used in humans, they are preferably combined with constant regions of human antibodies.

A chemical or biological modification may be performed on the anti-tetranor-PGDM monoclonal antibody as long as a desired effect is obtained. Chemical modification components include attachment of chemical moieties to the amino acid framework, chemical modification components of N-linked or O-linked carbohydrate chains and the like. Biological modification components may include post-translational modifications (for example, N-linked or O-linked glycosylation, N-terminal or C-terminal processing, deamidation, aspartic acid isomerization, and methionine oxidation) and addition of a methionine residue to the N terminal by expression using a prokaryotic host cell.

Modification components may further include deletion components in which one or two amino acids are deleted at the heavy chain carboxyl terminal and deletion components that are amidated.

The two heavy chains constituting the anti-tetranor-PGDM monoclonal antibody may include a combination of a full length chain and any one heavy chain selected from the group consisting of the above deletion components or a combination of any two heavy chains.

As used herein, a "fragment" of an antibody is a functional fragment that exhibits at least a part of the function exhibited by the antibody before it is fragmented. Examples thereof include Fab, $F(ab')_2$, scFv, Fab', and single chain antibodies (scFv). The fragment is not limited to these molecules, and may be any fragment as long as it specifically binds to tetranor-PGDM. For example, the anti-tetranor-PGDM monoclonal antibody may be an antibody having a single heavy chain variable region and having no light chain sequence such as a single domain antibody or nanobody.

The anti-tetranor-PGDM monoclonal antibody or a fragment thereof may comprise a linker depending on the purpose, and for example, when a plurality of variable regions constituting an antigen-binding site of an antibody are bound as in a single chain antibody, a linker may be interposed therebetween. For example, a single chain antibody is obtained by linking a heavy chain variable region and a light chain variable region via a linker. The linker sequence can be appropriately determined by those skilled in the art.

The fragment of the anti-tetranor-PGDM monoclonal antibody can be obtained by treating full-length molecules of antibody proteins with enzymes such as papain and pepsine. Alternatively, a gene encoding a desired fragment may be expressed in suitable host cells to produce the fragment. When host cells are transformed, the heavy chain sequence gene and the light chain sequence gene can be inserted into the same expression vector or can be inserted into separate expression vectors. Host cells may be either prokaryotic cells or eukaryotic cells. Examples of prokaryotic cells include bacterial cells such as *E. coli* and *bacillus subtilis.* When eukaryotic cells are used as hosts, animal cells, plant cells, and eukaryotic microorganisms can be used. Animal cells, plant cells, or fungal cells can be used. Examples of animal cells include the following cells.

(1) mammal cells: CHO, COS, myeloma, BHK, Hela, Vero, HEK293, Ba/F3, HL-60, Jurkat, SK-HEP1 and the like.

(2) amphibian cells: xenopus oocytes and the like.

(3) insect cells: sf9, sf21, Tn5 and the like.

(Polynucleotide)

In a second aspect, a polynucleotide encoding the anti-tetranor-PGDM monoclonal antibody or a fragment thereof of the present invention (polynucleotide of the present invention) is provided.

The polynucleotide of the present invention may be a single- or doublestranded polynucleotide (DNA including cDNA and the like or RNA including mRNA, cRNA and the like) including nucleotide sequence encoding an amino acid sequence of an anti-tetranor-PGDM monoclonal antibody or a fragment thereof and/or its complementary chain.

The heavy chain variable region (or heavy chain) and the light chain variable region (or light chain) of the anti-tetranor-PGDM monoclonal antibody or a fragment thereof may be encoded on the same polynucleotide, encoded on separate polynucleotides and provided as a combination thereof. The "polynucleotide encoding an anti-tetranor-PGDM monoclonal antibody or a fragment thereof" also includes a combination of a polynucleotide encoding a heavy chain

variable region (or heavy chain) and a polynucleotide encoding a light chain variable region (or light chain).

Examples of polynucleotides encoding an anti-tetranor-PGDM monoclonal antibody or a fragment thereof include those having a base sequence encoding at least one or more CDRs and preferably a base sequence encoding all six CDRs selected from the group consisting of the following:

(a) a base sequence encoding CDRH1 consisting of an amino acid sequence as shown in GYT-Xaa1-T-Xaa2-Y-Xaa3 (SEQ ID NO: 1), wherein Xaa1 is F, A or S, Xaa2 is N or R, and Xaa3 is A or W;

(b) a base sequence encoding CDRH2 consisting of an amino acid sequence as shown in I-Xaa4-P-Xaa5-Xaa6-Xaa7-Xaa8-T (SEQ ID NO: 2), wherein Xaa4 is N or Y, Xaa5 is F or G, Xaa6 is N or D, Xaa7 is D or G, and Xaa8 is G, N or D;

(c) a base sequence encoding CDRH3 consisting of an amino acid sequence as shown in A-Xaa9-Xaa10-Xaa11-Xaa12-Xaa13-Xaa14-Xaa15-Xaa16-Xaa17-Xaa18-Xaa19-Xaa20 (SEQ ID NO: 3), wherein Xaa9 is R, S or T, Xaa10 is Y or V, Xaa11 is Y or absent, Xaa12 is Y or absent, Xaa13 is Y or absent, Xaa14 is G or S, Xaa15 is S or R, Xaa16 is K or W, Xaa17 is A or F, Xaa18 is M or F, Xaa19 is D or A, and Xaa20 is Y or V;

(d) a base sequence encoding CDRL1 consisting of an amino acid sequence as shown in Xaa21-GAV-Xaa22-Xaa23-S-Xaa24-Xaa25 (SEQ ID NO: 4), wherein Xaa21 is T or S, Xaa22 is T or R, Xaa23 is T or I, Xaa24 is N, Y or K, and Xaa25 is Y or H;

(e) a base sequence encoding CDRL2 consisting of an amino acid sequence as shown in Xaa26-T-Xaa27, wherein Xaa26 is G or D, and Xaa27 is N or D; and

(f) a base sequence encoding CDRL3 consisting of an amino acid sequence as shown in ALW-Xaa28-SNHW-Xaa29 (SEQ ID NO: 5), wherein Xaa28 is Y, F or N, and Xaa29 is V or I.

Examples of VH base sequences including the base sequences (a) to (c) include SEQ ID NOs: 8, 21, 34, 47, 60, and 73. Examples of VL base sequences including the base sequences (d) to (f) include SEQ ID NOs: 15, 28, 41, 54, 67, and 80. A polynucleotide encoding a gene for an antibody or a fragment thereof may have substantially the same base sequence as a base sequence shown in any of the above SEQ ID Nos. Substantially the same base sequence means one, in a polynucleotide composed of a sequence having a homology of 80% or more, preferably an identity of 80% or more, for example, 81%, 82%, 83%, 84%, 85%, 86%, 87%, 88%, 89%, 90%, 91%, 92%, 93%, 94%, 95%, 96%, 97%, 98%, 99% or more, and preferably an identity of 99% or more, with respect to the base sequence shown in each SEQ ID NO, or alternatively in a polynucleotide that can hybridize under stringent conditions with a polynucleotide composed of a sequence complementary to a base sequence shown in each SEQ ID NO, a protein encoded with the polynucleotide has a desired function, for example, specifically binding to tetranor-PGDM.

Here, stringent conditions are hybridization conditions that are easily determined by those skilled in the art, and are generally empirical experiment conditions depending on the base length of the nucleic acid, the washing temperature, and the salt concentration. Generally, when the base is longer, the temperature for appropriate annealing is higher, and when the base is shorter, the temperature is lower. Hybrid formation generally depends on the ability of reannealing of the complementary chain in an environment of which the temperature is slightly lower than the melting point.

In one embodiment, a polynucleotide encoding an anti-tetranor-PGDM monoclonal antibody or a fragment thereof comprises the following:

(a) a base sequence encoding CDRH1 consisting of an amino acid sequence as shown in GYT-Xaa1-T-Xaa2-Y-Xaa3 (SEQ ID NO: 1), wherein Xaa1 is F, A or S, Xaa2 is N or R, and Xaa3 is A or W;

(b) a base sequence encoding CDRH2 consisting of an amino acid sequence as shown in I-Xaa4-P-Xaa5-Xaa6-Xaa7-Xaa8-T (SEQ ID NO: 2), wherein Xaa4 is N or Y, Xaa5 is F or G, Xaa6 is N or D, Xaa7 is D or G, and Xaa8 is G, N or D; and

(c) a base sequence encoding CDRH3 consisting of an amino acid sequence as shown in A-Xaa9-Xaa10-Xaa11-Xaa12-Xaa13-Xaa14-Xaa15-Xaa16-Xaa17-Xaa18-Xaa19-Xaa20 (SEQ ID NO: 3), wherein Xaa9 is R, S or T, Xaa10 is Y or V, Xaa11 is Y or absent, Xaa12 is Y or absent, Xaa13 is Y or absent, Xaa14 is G or S, Xaa15 is S or R, Xaa16 is K or W, Xaa17 is A or F, Xaa18 is M or F, Xaa19 is D or A, and Xaa20 is Y or V,

or may comprise a base sequence encoding CDRH1, CDRH2 and CDRH3 composed of a base sequence

having deletion, substitution or addition of one or several bases in the base sequence encoding each CDR,

or may comprise a base sequence encoding CDRH1, CDRH2 and CDRH3 composed of a base sequence with at least 80% identity to a base sequence encoding each CDR.

In one embodiment, a polynucleotide encoding an anti-tetranor-PGDM monoclonal antibody or a fragment thereof comprises the following:

(d) a base sequence encoding CDRL1 consisting of an amino acid sequence as shown in Xaa21-GAV-Xaa22-Xaa23-S-Xaa24-Xaa25 (SEQ ID NO: 4), wherein Xaa21 is T or S, Xaa22 is T or R, Xaa23 is T or I, Xaa24 is N, Y or K, and Xaa25 is Y or H;

(e) a base sequence encoding CDRL2 consisting of an amino acid sequence as shown in Xaa26-T-Xaa27, wherein Xaa26 is G or D, and Xaa27 is N or D; and

(f) a base sequence encoding CDRL3 consisting of an amino acid sequence as shown in ALW-Xaa28-SNHW-Xaa29 (SEQ ID NO: 5), wherein Xaa28 is Y, F or N, and Xaa29 is V or I,

or may comprise a base sequence encoding CDRL1, CDRL2 and CDRL3 composed of a base sequence having deletion, substitution or addition of one or several bases in the base sequence encoding each CDR,

or may comprise a base sequence encoding CDRL1, CDRL2 and CDRL3 composed of a base sequence encoding each CDR and a base sequence having an identity of 80% or more.

In one embodiment, a polynucleotide encoding a gene for an anti-tetranor-PGDM monoclonal antibody or a fragment thereof comprises the following:

(a) a base sequence encoding CDRH1 consisting of an amino acid sequence as shown in GYT-Xaa1-T-Xaa2-Y-Xaa3 (SEQ ID NO: 1), wherein Xaa1 is F, A or S, Xaa2 is N or R, and Xaa3 is A or W;

(b) a base sequence encoding CDRH2 consisting of an amino acid sequence as shown in I-Xaa4-P-Xaa5-Xaa6-Xaa7-Xaa8-T (SEQ ID NO: 2), wherein Xaa4 is N or Y, Xaa5 is F or G, Xaa6 is N or D, Xaa7 is D or G, and Xaa8 is G, N or D;

(c) a base sequence encoding CDRH3 consisting of an amino acid sequence as shown in A-Xaa9-Xaa10-Xaa11-Xaa12-Xaa13-Xaa14-Xaa15-Xaa16-Xaa17-Xaa18-Xaa19-Xaa20 (SEQ ID NO: 3), wherein Xaa9 is R, S or T, Xaa10 is Y or V, Xaa11 is Y or absent, Xaa12 is Y or absent, Xaa13 is Y or absent, Xaa14 is G or S, Xaa15 is S or R, Xaa16 is K or W, Xaa17 is A or F, Xaa18 is M or F, Xaa19 is D or A, and Xaa20 is Y or V;

(d) a base sequence encoding CDRL1 consisting of an amino acid sequence as shown in Xaa21-GAV-Xaa22-Xaa23-S-Xaa24-Xaa25 (SEQ ID NO: 4), wherein Xaa21 is T or S, Xaa22 is T or R, Xaa23 is T or I, Xaa24 is N, Y or K, and Xaa25 is Y or H;

(e) a base sequence encoding CDRL2 consisting of an amino acid sequence as shown in Xaa26-T-Xaa27, wherein Xaa26 is G or D, and Xaa27 is N or D; and

(f) a base sequence encoding CDRL3 consisting of an amino acid sequence as shown in ALW-Xaa28-SNHW-Xaa29 (SEQ ID NO: 5), wherein Xaa28 is Y, F or N, and Xaa29 is V or I,

or may comprise a base sequence encoding CDRH1, CDRH2 and CDRH3 and CDRL1, CDRL2 and CDRL3 composed of a base sequence having deletion, substitution or addition of one or several bases in the base sequence encoding each CDR,

or may comprise a base sequence encoding CDRH1, CDRH2 and CDRH3 and CDRL1, CDRL2 and CDRL3 composed of a base sequence with at least 80% identity to a base sequence encoding each CDR.

(Vector and host cell)

In a third aspect, an expression vector containing a polynucleotide encoding an anti-tetranor-PGDM monoclonal antibody or a fragment thereof and host cells transfected with the expression vector are provided. The host cells transfected with the expression vector can produce an anti-tetranor-PGDM monoclonal antibody or a fragment thereof.

A polynucleotide encoding a heavy chain variable region (or heavy chain) of an anti-tetranor-PGDM monoclonal antibody or a fragment thereof and a polynucleotide encoding a light chain variable region (or light chain) of an anti-tetranor-PGDM monoclonal antibody or a fragment thereof may be contained in the same expression vector or may be incorporated into separate expression vectors and provided as a combination. The "expression vector containing a polynucleotide encoding an anti-tetranor-PGDM monoclonal antibody or a fragment thereof" also include a combination of an expression vector containing a polynucleotide encoding a heavy chain variable region (or heavy chain) (expression vector of a heavy chain variable region (or heavy chain)) and an expression vector containing a polynucleotide encoding a light chain variable region (or light chain) (expression vector of a light chain variable region (or light chain)).

An expression vector containing a polynucleotide encoding an anti-tetranor-PGDM monoclonal antibody or a fragment thereof and host cells transfected with the expression vector can be obtained by a method known to those skilled in the art. For example, a polynucleotide encoding a single-chain anti-tetranor-PGDM monoclonal antibody is incorporated into a vector suitable for its expression and introduced into hosts, and thus the anti-tetranor-PGDM monoclonal antibody or a fragment thereof is expressed. The expression vector preferably contains a promoter, a terminator and the like suitable for expressing an anti-tetranor-PGDM monoclonal antibody or a fragment thereof in host cells. The expression vector can further contain other appropriate control sequences such as an enhancer.

The vector can be appropriately selected from among expression vectors well-known to those skilled in the art in consideration of the type of host cells and the expression efficiency of the anti-tetranor-PGDM monoclonal antibody or a fragment thereof, and the type may be a plasmid vector or of a virus vector derived from retrovirus or adenovirus.

Host cells are not limited as long as they express an anti-tetranor-PGDM monoclonal antibody or a fragment thereof from an expression vector, and include prokaryotic cells such as *E. coli,* mammal-derived cells such as CHO, COS, NIH3T3, HEK293, HEK293T, and COS-7, insect-derived cells such as F9, and eukaryotic cells such as yeast cells. Examples of mammals include primates such as humans and chimpanzees and rodents such as mice, rats, and hamsters.

Transfection of the expression vector into host cells can be performed using, for example, a method well-known to those skilled in the art such as an electroporation method, a calcium phosphate method, a lipofection method, or a DEAE dextran method, or a method using a commercially available reagent.

The host cells (transfectants) transfected with an expression vector containing a polynucleotide encoding an anti-tetranor-PGDM monoclonal antibody or a fragment thereof include

- a transfectant that contains the expression vector and expresses an anti-tetranor-PGDM monoclonal antibody or a fragment thereof; and

- a transfectant (stable transfectant) that expresses an anti-tetranor-PGDM monoclonal antibody or a fragment thereof by incorporating a polynucleotide encoding an anti-tetranor-PGDM monoclonal antibody or a fragment thereof into the chromosome of host cells in an expressible manner. Such stable transfectants may or may not contain an expression vector containing a polynucleotide encoding an anti-tetranor-PGDM monoclonal antibody or a fragment thereof. Transfectants are preferably mammal-derived cells such as CHO, COS, NIH3T3, HEK293, HEK293T, and COS-7.

(Hybridoma)

In a fourth aspect, a hybridoma that produces an anti-tetranor-PGDM monoclonal antibody is provided.

Hybridomas producing an anti-tetranor-PGDM monoclonal antibody can be obtained by immunizing an animal to be immunized with tetranor-PGDM according to a general method and fusing immune cells obtained from the immunized animal with known myeloma cells according to a general cell fusion method. However, tetranor-PGDM has a low-molecular weight, and is also present in vivo, and thus antibodies are unlikely to be produced. Therefore, it is preferable to use mice in which genes encoding two types of $PGD_2$ synthases, a hematopoietic and lipocalin type, are deleted by a gene modification technique, and which do not produce $PGD_2$ or its metabolite tetranor-PGDM, or the like as an animal to be immunized, and conjugate the immunogen tetranor-PGDM with a carrier protein such as keyhole limpet hemocyanin (KLH). Mice that do not produce $PGD_2$ or its metabolite tetranor-PGDM can be obtained by, for example, replacing cysteine 65 in the $PGD_2$ production site present in L-PGDS exon II with serine using a technique such as a CRISPR/Cas system. The 65th cysteine in the amino acid sequence (SEQ ID NO: 84) of L-PGDS is the center of enzyme activity (J Biol Chem. 2009 14; 284(33): 22344-52.), and if this cysteine is replaced with other amino acids, for example, serine,

the PGD synthase activity disappears and $PGD_2$ cannot be produced. Cells producing an anti-tetranor-PGDM monoclonal antibody are screened from among such hybridomas. Examples of animals to be immunized include rodents such as mice, rats, hamsters, and rabbits, and mammals such as monkeys.

(Method of producing anti-tetranor-PGDM monoclonal antibody)

In a fifth aspect, a method of producing an anti-tetranor-PGDM monoclonal antibody or a fragment thereof is provided. An anti-tetranor-PGDM monoclonal antibody or a fragment thereof may be produced by culturing the above host cells, transfectants and hybridomas or produced using a recombinant DNA technique.

Examples of antibodies produced as recombinant proteins by a recombinant DNA technique include chimeric antibodies, humanized antibodies and human antibodies. These antibodies can be produced, for example, by culturing host cells transformed with a recombinant vector encoding the antibody or its heavy chain or light chain. Recombinant antibodies can also be produced using transgenic animals into which a gene encoding a desired antibody is introduced.

Culture conditions for host cells, transfectants and hybridomas can be appropriately determined by those skilled in the art according to a desired anti-tetranor-PGDM monoclonal antibody or a fragment thereof and the type of host cells. The production method may further include a step of recovering an antibody or a fragment thereof that specifically binds to tetranor-PGDM from the cultured product obtained in the culturing step.

The produced antibody or fragment thereof may be further subjected to an additional step such as an additional purifying step before recovery, and for example, in order to increase purity, it is preferable to perform salting-out, filter filtration, clarification using centrifugation, recovery by affinity, intermediate purification by ion exchange, and final purification by gel filtration.

(Diagnostic agent for tetranor-PGDM-related disease)

In a sixth aspect, various applications of anti-tetranor-PGDM monoclonals, for example, diagnostic agents for tetranor-PGDM-related disease, comprising the antibody or fragment thereof, are provided.

**As** used herein, "tetranor-PGDM-related disease" means a disease for which tetranor-PGD serves as a diagnostic marker such as a food allergy (WO 2016/021704 (mentioned above); Maeda S, Nakamura T, Harada H, Tachibana Y, Aritake K, Shimosawa T, et al. Prostaglandin D2 metabolite in urine is an index of food allergy. Sci Rep. 2017; 7(1): 17687. Epub 2017/12/17. doi: 10.1038/s41598-017-17798-w. PubMed PMID: 29247205; PubMed Central PMCID: PMCPMC5732293.; Inagaki S, Maeda S, Narita M, Nakamura T, Shimosawa T, Murata T, et al. Urinary PGDM, a prostaglandin D2 metabolite, is a novel biomarker for objectively detecting allergic reactions of food allergy. J Allergy Clin Immunol. 2018; 142(5): 1634-6.e10. Epub 2018/07/10. doi: 10.1016/j.jaci.2018.06.032. PubMed PMID: 29981807.), anaphylactic reaction, muscular dystrophy such as Duchenne muscular dystrophy (Takeshita E, Komaki H, Tachimori H, Miyoshi K, Yamamiya I, Shimizu-Motohashi Y, et al. Urinary prostaglandin metabolites as Duchenne muscular dystrophy progression markers. Brain Dev. 2018; 40(10): 918-25. Epub 2018/07/15. doi: 10.1016/j.braindev.2018.06.012. PubMed PMID: 30006121.), and aspirin intolerant asthma (Higashi et al., "Urinary tetranor-PGDM concentrations in aspirin-intolerant asthma and anaphylaxis", J Allergy Clin Immunol. 2012 Feb; 129(2): 557-9, 559.e1-2. doi: 10.1016/j.jaci.2011.09.019. Epub 2011 Oct 22.).

(Kit comprising anti-tetranor-PGDM monoclonal antibody)

In a seventh aspect, a kit comprising an anti-tetranor-PGDM monoclonal antibody or a fragment thereof is provided.

The kit is intended to be used for tetranor-PGDM measurement, for example, a qualitative test or quantitative test. As a method of measuring tetranor-PGDM, for example, an immunoassay using an antigen-antibody reaction with tetranor-PGDM in a specimen is preferable.

For the immunoassay, a detectably labeled anti-tetranor-PGDM monoclonal antibody or an antibody (secondary antibody) to a detectably labeled anti-tetranor-PGDM monoclonal antibody is used. Depending on the antibody labeling method, methods are classified into enzyme immunoassay (EIA or ELISA), radioimmunoassay (RIA), fluoroimmunoassay (FIA), fluorescence polarization immunoassay (FPIA), chemiluminescence immunoassay (CLIA) and the like, and any of these can be suitably used in the kit. The above measurement methods are only examples, and the above antibodies can also be used in other known methods known to those skilled in the art, for example, an aggregation method, chemiluminescence immunoassay (CLIA), electrochemiluminescence immunoassay (ECLIA) and the like.

Antibodies labeled with enzymes such as peroxidase and alkaline phosphatase are used in the ELISA method, antibodies labeled with radioactive substances such as $^{125}I$, $^{131}I$, $^{35}S$, and $^{3}H$ are used in the RIA method, antibodies labeled with fluorescent substances such as fluorescein isothiocyanate, rhodamine, dansyl chloride, phycoerythrin, tetramethylrhodamine isothiocyanate, and near-infrared fluorescent materials are used in the FPIA method, and antibodies labeled with luminescent substances such as luciferase, and luciferin, aequorin are used in the CLIA method. In addition, antibodies labeled with nanoparticles such as colloidal gold, and quantum dots can be detected.

In addition, in the immunoassay, an anti-tetranor-PGDM monoclonal antibody can be labeled with biotin and bound with avidin or streptavidin labeled with an enzyme or the like for detection.

Among immunoassays, in an ELISA method using an enzyme label, it is possible to measure an antigen easily and rapidly. The ELISA method includes a competitive method and a sandwich method. In the competitive method, an anti-tetranor-PGDM monoclonal antibody is immobilized on a solid phase carrier such as a microplate, and a urine specimen and an enzyme-labeled tetranor-PGDM are added to induce an antigen-antibody reaction. After washing once, a reaction with an enzyme substrate is performed to develop a color and the absorbance is measured. When the level of tetranor-PGDM in the urine specimen is higher, the color development becomes weaker, and when the tetranor-PGDM level in the urine specimen is lower, the color development becomes stronger, and thus the tetranor-PGDM level can be determined using a calibration curve.

In the sandwich method, an anti-tetranor-PGDM monoclonal antibody is immobilized on a solid phase carrier, a urine sample is added and reacted, and another enzyme-labeled anti-tetranor-PGDM monoclonal antibody is then additionally added and reacted. After washing, a reaction with an enzyme substrate is performed to develop a color, the absorbance is measured, and thus the tetranor-PGDM level can be determined. In the sandwich method, after the antibody immobilized on the solid phase carrier and tetranor-PGDM in the urine specimen are reacted, an unlabeled anti-tetranor-PGDM monoclonal antibody (primary antibody) is first added, and an antibody (secondary antibody) to this unlabeled anti-tetranor-PGDM component may be labeled with an enzyme and then additionally added.

When the enzyme is a peroxidase, 3,3'-diaminobenzidine (DAB), 3,3',5,5'-tetramethylbenzidine (TMB), o-phenylenediamine (OPD) or the like can be used as the enzyme substrate, and when the enzyme is an alkaline phosphatase, p-nitropheny phosphate (NPP) or the like can be used as the enzyme substrate.

**As** used herein, "solid phase carrier" is not particularly limited as long as it can immobilize an antibody, and examples thereof include microtiter plates made of glass, a metal, a resin or the like, substrates, beads, nitrocellulose membranes, nylon membranes, and PVDF membranes, and target substances and antibodies can be immobilized on these solid phase carriers according to a known method.

Although the form of the kit is not limited, a form of a stick comprising an anti-tetranor-PGDM monoclonal antibody and in which the tetranor-PGDM level in the specimen can be visualized with a colored line or the like is preferable. A known kit can be used for such a stick-like kit. For example, a stick using an immunochromatographic method is known, and a configuration in which an antibody storage unit in which a first anti-tetranor-PGDM monoclonal antibody labeled with colloidal gold or the like is stored and a determination unit in which a second anti-tetranor-PGDM monoclonal antibody that recognizes another epitope of tetranor-PGDM is immobilized in a line on a cellulose membrane or the like are connected via a narrow groove can be used. When a specimen is added to such a stick, the labeled antibody and tetranor-PGDM are bound in the antibody storage unit to form a tetranor-PGDM-labeled antibody complex, and the complex moves to the determination unit through the groove due to a capillary phenomenon. When the complex is captured by the immobilized second anti-tetranor-PGDM monoclonal antibody, a red line appears in the determination unit due to a plasmon effect of the colloidal gold.

A control line on which an antibody to the labeled antibody is immobilized may be provided in the determination unit of the stick. Since the control line develops a color with or without tetranor-PGDM, it is possible to check whether the test is performed correctly.

The stick may further comprise absorbent paper for absorbing a urine specimen, a desiccant and the like.

In the stick, the labeled anti-tetranor-PGEM antibody is also stored in the antibody storage unit, and the anti-tetranor-PGEM antibody that recognizes another epitope of tetranor-PGEM is immobilized in a line in the determination unit, and thus tetranor-PGDM and tetranor-PGEM can be measured at the same time.

With such a stick, the tetranor-PGDM level can be easily measured even at home, and the risk of developing a food allergy and its degree can be evaluated.

The kit can be used not only for diagnosing a tetranor-PGDM-related disease, for example, a food allergy or Duchenne muscular dystrophy, but also for evaluating activation of mast cells in a food allergy.

(Method for detecting tetranor-PGDM)

In an eighth aspect, a method for detecting tetranor-PGDM using an anti-tetranor-PGDM monoclonal antibody or a fragment thereof is provided.

The method for detecting tetranor-PGDM can be used not only for diagnosing a food allergy or Duchenne muscular dystrophy but also for evaluating activation of mast cells in a food allergy.

(Method for diagnosing food allergy)

In a ninth aspect, a method for diagnosing a food allergy using an anti-tetranor-PGDM monoclonal antibody or a fragment thereof is provided.

The urinary tetranor-PGDM level increases according to the increase in severity of food allergy symptoms, and the number and activation of mast cells in tissues, and decreases according to inhibition of symptoms by administration of a food allergy treatment agent. Therefore, when the urinary tetranor-PGDM level is measured, it is possible to evaluate not only whether a subject has a food allergy but also its severity. In addition, in the urine of the subject with a food allergy, a high tetranor-PGDM concentration may be observed without performing an antigen stimulation. Therefore, it is possible to measure the urinary tetranor-PGDM level in the subject who has not developed a food allergy and to evaluate a risk of developing a food allergy.

Such an evaluation method may comprise a step of detecting the urinary tetranor-PGDM level and a step of assisting in determining that, when the urinary tetranor-PGDM level is higher, food allergy symptoms are severe or have become severe, or a risk of developing symptoms is higher or has become higher. The threshold value used in such determination may vary depending on factors such as a subject age. Therefore, although not intended to be limiting, the urinary tetranor-PGDM concentration is $1.5 \pm 0.3$ ng/mg creatinine (Cre) in healthy individuals, but the urinary tetranor-PGDM concentration may be 2.25 ng/mg Cre or more in subjects who have or have developed severe food allergy symptoms or who are or have become at high risk of developing symptoms.

**As** used herein, "food allergy" refers to various allergy reactions that occur when allergens contained in food are taken into the body. As food allergies, allergies to chicken eggs, milk, crustaceans, wheat, fruit, nuts, seafood, buckwheat and the like are well known, but allergies to allergens other than these food components are also included. Symptoms occur in the skin, mucous membranes, digestive organs, respiratory organs and the like and are typically diarrhea, vomiting, skin inflammation and the like.

As used herein, "subject" includes a person who may have a food allergy, a person who has actually developed a food allergy, a person who is undergoing a food allergy treatment, a person who is taking a food allergy treatment agent, a person who is unsure of whether he/she has a food allergy and the like. In addition, "subject" is not limited to humans, and may be mammals such as mice, rats, rabbits, cats, dogs, monkeys, pigs, sheep, cows, and horses.

In one aspect of the food allergy test method according to the present invention, when the urinary tetranor-PGDM level is higher, it is determined that symptoms are more severe or a development risk is higher. Regarding whether the urinary tetranor-PGDM level is higher, the tetranor-PGDM level may be determined by measuring the tetranor-PGDM levels in urine collected from the same individual a plurality of times and comparing the results. In this case, it is possible to evaluate changes in the severity and the development risk in the same individual. In addition, whether the urinary tetranor-PGDM level is higher may be determined by measuring the tetranor-PGDM levels in urine collected from a plurality of persons and comparing the results. In this case, it is possible to evaluate whether the severity or the development risk is higher than those of other people.

In one aspect of the food allergy test method according to the present invention, when the urinary tetranor-PGDM level is higher than a predetermined value, it is determined that symptoms are severe or a development risk is high, and when the level is lower than a predetermined value, it is determined that symptoms are mild or a development risk is low. The predetermined value (cutoff value) can be determined by comparing the tetranor-PGDM level in urine specimens of a plurality of healthy subjects or non-food allergy patients with the tetranor-PGDM level in urine specimens of a plurality of food allergy patients by a general method.

In one aspect, the food allergy test method according to the present invention is used to evaluate a treatment agent administered to food allergy patients and a treatment method to be performed on patients. For example, the tetranor-PGDM level in the urine specimen collected at any time between before the treatment starts and after the treatment starts is measured, and when the tetranor-PGDM level decreases, it is determined that the treatment agent or the treatment method is effective, and when the tetranor-PGDM level does not decrease but increases, it is determined that the treatment agent or the treatment method is not effective. In this case, the decrease or increase in the tetranor-PGDM level may not be a significant decrease or increase, and may be to the extent that those skilled in the art can determine that there is a tendency to decrease or increase.

In one aspect, the food allergy test method according to the present invention may be used to evaluate the effect of agents in animal experiments for development of food allergy treatment agents.

In one aspect, the food allergy test method according to the present invention involves examining a sample collected from a subject in order to obtain information necessary for diagnosis. Such a test method is performed by, for example, a test company.

For urine to be used in the test of the present invention, a sample collected by a general method can be used. It may be a single urine collection or two or more urine collections. The collected urine may be stored at room temperature during the test or stored at -40°C or lower, for example, -80°C. Frozen urine can be rapidly thawed and used for measurement.

In addition to the urinary tetranor-PGDM level in subjects, the level of tetranor-PGEM (9,15-dioxo-11α-hydroxy-13,14-dihydro-2,3,4,5-tetranor-prostan-1,20-dioic acid) which is a metabolite of $PGE_2$, may be measured.

**The** urinary tetranor-PGDM level behaves differently in a food allergy than in other inflammatory diseases. Therefore, when the urinary tetranor-PGDM level is measured, it is possible to distinguish symptoms from other inflammatory diseases (for example, enteritis, asthma, allergic dermatitis). In addition, the urinary tetranor-PGEM level also behaves differently in a food allergy than in other inflammatory diseases. Therefore, when both the urinary tetranor-PGDM level and the urinary tetranor-PEGM level are measured, it is possible to distinguish symptoms more accurately.

More specifically, when the urinary tetranor-PGDM level remains elevated for a certain period of time and the urinary tetranor-PGEM level transiently increases and then immediately decreases, it can be determined that there is a food allergy. Here, a certain period of time is, for example, 3 days or longer, 5 days or longer, 7 days or longer, 10 days or longer, or 20 days or longer after a substance that causes a food allergy is taken. Transiently increasing and then immediately decreasing means, for example, decreasing within 5 days, 4 days, or 3 days after increasing.

The urinary tetranor-PGEM level can be measured using a known method, for example, a mass spectrometer.

Activation of mast cells in a food allergy can be evaluated using an anti-tetranor-PGDM monoclonal antibody or a fragment thereof.

As used herein, "activation of mast cells" means an increase in the number of mast cells and an increase in activities including degranulation. As shown in examples to be described below, the urinary tetranor-PGDM level increases as the number of mast cells increases, but does not increase under inhibition of degranulation. In addition, the urinary tetranor-PGEM level also increases as the number of mast cells increases. Therefore, activation of mast cells can be evaluated using the urinary tetranor-PGDM level and the urinary tetranor-PGEM level as indicators.

For example, it is known that allergic rhinitis causes symptoms in mast cell-deficient mice, but a food allergy causes almost no symptoms in mast cell-deficient mice, and it is known that a food allergy is highly dependent on mast cells, and an increase in the number of mast cells and activities thereof are important for the development and progress of a food allergy. As a method for detecting activation of the mast cells (particularly gastrointestinal mucosal mast cells) specific to a food allergy, the only method is to measure the blood histamine concentration so far. However, since blood histamine disappears in 15 minutes, there is no actual method for detecting activation of mast cells.

(Method for treating tetranor-PGDM-related diseases)

In a tenth aspect, a method of treating a subject diagnosed with an anti-tetranor-PGDM-related disease is provided.

A necessary treatment is appropriately performed on a subject diagnosed with a tetranor-PGDM-related disease according to the above diagnosis method. For example, a known treatment method such as a hyposensitization treatment can be applied to a subject diagnosed with a food allergy.

The hyposensitization treatment is performed to induce immune tolerance by administering dilute allergens to patients under the supervision of a doctor. Many allergic disease treatment methods are symptomatic treatments, but the hyposensitization treatment is being focused on because it targets radical curing by acting on the mechanisms of action of an allergic disease. The hyposensitization treatment begins with a very low dose and the dose gradually increases, but in this case, a method of appropriately evaluating the severity of allergic symptoms in patients is required in order to prevent severe symptoms from occurring due to administration of an amount of allergens exceeding a threshold value.

**The** administration route, dose and frequency of administration of the hyposensitization treatment are not particularly limited, and can be appropriately selected according to various conditions such as the patient's age, weight, and symptoms. Examples of administration routes include routes of administration to individuals by intradermal, subcutaneous, intramuscular, intraperitoneal, transdermal, transmucosal, oral, and inhalation routes.

While the present invention will be described below in more detail with reference to examples, the present invention is not limited thereto.

## Examples

### Material and method

#### Mice

Using an offset-nicking method to which a CRISPR/Cas system was applied, L-PGDS C65S mutant-mice in which cysteine at position 65 of L-PGDS (SEQ ID NO: 84) was replaced with serine were prepared. Specifically, using exon-3 of L-PGDS of targeted embryonic stem cells as a targeting vector, after cleavage using Cas9 nickase and guide RNA, vector DNA was inserted to convert TGC (cysteine) to AGC (serine). L-PGDS C65S mutant mice were obtained

by microinjection of these cells into 8-cell stage embryo of ICR strain mice.

Preparation of monoclonal antibody

Keyhole limpet hemocyanin (KLH) conjugated tetranor-PGDM was subcutaneously administered to L-PGDS C65S mice to perform immunization. Splenocytes of the immunized mice were fused with myeloma cells (SP2/0-Ag14) according to standard protocols [6]. Positive hybridomas were cloned by a limiting dilution method. IgG antibodies were purified from serum-free media of the hybridoma culture supernatant.

Determination of cDNA sequence of variable region of each antibody

Isolation of gene sequence encoding VH and VL of anti-tetranor-PGDM monoclonal antibodies

Total RNA was prepared from $1\times10^6$ hybridomas producing each antibody using TRI Reagent (Cat#TR118, commercially available from Molecular Research Center, Inc.).

Gene cloning of VH and VL of anti-tetranor-PGDM monoclonal antibody: cDNA was prepared from 1 μg of total RNA of each hybridoma using SMARTer (registered trademark) Mouse BCR IgG H/K/L Profiling Kit (Cat#634422, commercially available from Clontech). The isotype of the obtained antibody was identified using Iso-Gold Rapid Mouse Monoclonal Antibody Isotyping κ&λ Kit (Cat# KSOT03-010, commercially available from BioAssay Works). Using the obtained cDNA as a template, BCR Primer 1V (containing a forward primer) bundled in the kit, and a reverse primer of mBCR Primer 1H (encoding a mouse IgG heavy chain constant region), mBCR Primer 1L(κ) or mBCR Primer 1L(λ) (encoding a mouse IgG light chain constant region) were used in combination, and thus cDNA sequences of VH and VL were determined.

Specifically, a PCR reaction was performed using a combination of BCR Primer 1V and mBCR Primer 1H, and VH cDNA fragments of the antibodies were amplified. In addition, PCR was performed using a combination of mouse Ig(κ)-specific primer (mBCR Primer 1L(κ)) and BCR Primer 1V or a combination of mouse Ig(λ)-specific primer (mBCR Primer 1L(λ)) and BCR Primer 1V, and VL cDNA fragments of the antibodies were amplified.

PCR was performed for 35 reaction cycles including 98°C for 10 seconds, 60°C for 15 seconds, and 68°C for 45 seconds.

Each PCR amplification product was purified using FastGene Gel/PCR Extraction kit (Cat#FG-91202, commercially available from NIPPON Genetics Co., Ltd.).

dA was added to the 3' terminal of the obtained gene fragment using TArget CloneTM -Plus-(Cat#TAK-201, commercially available from TOYOBO) and inserted onto the pTA2 Vector bundled in the kit.

The obtained plasmid was introduced into *E. coli* DH5α stains. A plasmid was extracted from the obtained transformant using FastGene Plasmid Mini Kit (Cat#FG-90502, commercially available from NIPPON Genetics Co., Ltd.) and the base sequence was analyzed. As a result, it was confirmed that the full-length VH cDNA and VL cDNA having an ATG sequence assumed to be the start codon at the 5' terminal of cDNA were obtained.

Competitive enzyme immunoassay method

The reactivity of the tetranor-PGDM monoclonal antibody was examined by competitive EIA. Tetranor-PGDM monoclonal antibodies with various concentrations were incubated in an anti-mouse goat IgG antibody-immobilized 96-well plate (Cayman Chemical, Michigan, USA) for 1 hour. Next, the plate was washed three times with a phosphate buffered saline (PBS-T) containing 0.5% bovine serum albumin (BSA) and 0.05% (v/v) Tween-20 (PBS-T). Subsequently, a tetranor-PGDM-conjugated acetylcholinesterase tracer (tracer) was incubated overnight. Next, the plate was washed three times with PBS-T and incubated with an Ellman's reagent for 1 hour. The absorbance at 405 nm was measured with a microplate reader (Perkin Elmer, Massachusetts, USA). The selectivity of tetranor-PGDM with respect to a prostaglandin metabolite was examined by competitive EIA using tetranor-PGEM, tetranor-PGAM, and tetranor-PGFM.

**The** results are shown below.

[Table 1]

| **Antibody** | IC50 (ng/mL) |
|---|---|
| #31-13 | 4.8 |
| #44-63 | 25.6 |
| #47-11 | 33.5 |
| #50-32 | 22.7 |

(continued)

| **Antibody** | IC50 (ng/mL) |
|---|---|
| #53-113 | 418.8 |
| #60-141 | 1.79 |

Development of EIA method

The influence of the pH (5.0, 6.0, 6.5, 7.0, 7.5, 8.0, and 9.0) and the ionic strength (NaCl content in a 10 mM phosphate buffer solution) on the EIA performance was examined. A standard inhibition curve of tetranor-PGDM was plotted under each condition. The cross-reactivity was calculated based on the following formula.

$$\text{cross-reactivity (\%)}=(\text{IC}_{50}\text{ of tetranor-PGDM/IC}_{50}\text{ of other tetranor-PGM})\times 100.$$

Reproducibility test

Tetranor-PGDM with a known concentration was added to the assay solution for measurement. Simultaneous reproducibility was obtained from 9 measurements, and inter-day reproducibility was obtained by performing measurement for 3 days.

Accuracy prediction

A data set of the analyte concentration and the absorbance was fitted with the following 4-parameter logistic formula with fitting parameters a, b, c, and d. [7, 8]

$$f(X)=(a-d)/(1+(X/c)^{b})+ d$$

Based on the model [9] proposed by Hayashi et al., the relative standard deviation (RSD) of the entire analysis ($\rho T$) was represented by the following formula.

$$\rho T^2= X^2/(X+G)^2\times(\rho X^2+\rho G^2)+\rho B^2+\rho S^2+(\sigma W/f(X)\times 100)^2 \text{ [9]}$$

In this formula, G was the amount of labeled antigens, $\rho X$, $\rho G$, $\rho B$, and $\rho S$ were RSDs of pipette volumes of analytes labeled as the antigen, the antibody, and the substrate and $\rho S$ was two thirds of the RSD of the chromogenic substrate solution of the pipette volume. $\sigma W$ was the SD of the intrinsic absorbance between wells of the plate. Since the concentration of labeled antigens was unknown, the approximate formula of the uncertainty of the competitive EIA $\rho T^2=\rho B^2+(\sigma W/f(X)\times 100)^2$ was used [9]. $\rho B$= 0.782116 and oW= 0.0026. $\rho T$ was calculated from the blank-subtracted measured value. For the $\rho B$ value, the weight of the liquid collected with a pipette was measured 20 times and the RSD was calculated. The oW value was calculated from the well-to-well SD of the absorbance of empty wells (n=96).

Measurement of tetranor-PGDM in artificial urine

Tetranor-PGDM dissolved in distilled water was added to artificial urine (Table 2) to prepare urine samples with four different concentrations (1.25, 2.5, 5.0, and 10.0 ng/mL).

[Table 2]

| | mM |
|---|---|
| $Na_2SO_4$ | 11.965 |
| $C_5H_4N_4O_3$ | 1.487 |
| $Na_3C_6H_5O_7.2H_2O$ | 2.450 |
| $C_4H_7N_3O$ | 7.791 |
| $CH_4N_2O$ | 249.750 |
| KCl | 30.953 |

(continued)

| | mM |
|---|---|
| NaCl | 30.053 |
| $CaCl_2$ | 1.663 |
| $NH_4Cl$ | 23.667 |
| $K_2C_2O_4.H_2O$ | 0.19 |
| $MgSO_4.7H_2O$ | 4.389 |
| $NaH_2PO_4.2H_2O$ | 18.667 |
| $Na_2HPO_4.2H_2O$ | 4.667 |

For solid phase extraction (SPE), 0.4 mL of a urine sample was diluted to 0.8 mL with 0.1% (v/v) formic acid. This was applied to an SPE cartridge column (HLBμ Elution plate, Waters, Massachusetts, USA) pretreated with 200 μL of acetonitrile and distilled water. After the column was washed with 200 μL of distilled water and 200 μL of hexane, the lipid fraction was eluted with 50 μL of acetonitrile. The eluate was recovered and dried in a vacuum, and the obtained residue was redissolved in 0.4 mL of an assay solution. Next, the sample solution containing tetranor-PGDM was subjected to EIA. At the same time, tetranor-PGDM was measured through LC-MS/MS [2, 3]. The recovery from SPE determined through LC-MS/MS was 77.1% (Table 3).

[Table 3]

| Sample | Area | | Recovery (%) | Mean Recovery (%) |
|---|---|---|---|---|
| | SPE | | | |
| | - | + | | |
| 1 | 9228 | 7844 | 85.0 | |
| 2 | 10937 | 7970 | 72.9 | 77.1 |
| 3 | 10215 | 7508 | 73.5 | |

Results

Optimization of competitive EIA

A hybridoma producing monoclonal antibodies against tetranor-PGDM (Mab) was obtained from the immunized mouse splenocytes. Next, 60-141Mab was purified and applied in a competitive EIA.

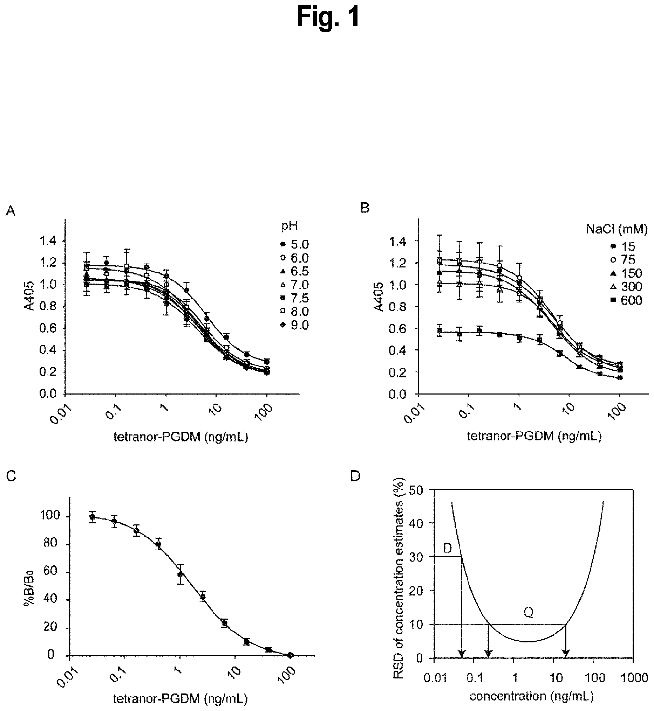

Since patient urine samples had different physicochemical properties, the optimal ionic strength and the pH for Mab-based EIA were examined. As shown in Fig. 1A, the 600 mM NaCl concentration strongly interfered with the competition curve. When the NaCl concentration in the assay buffer was 15, 75, 150, and 300 mM, the IC50 value was 6.6, 5.7, 5.3, and 7.3 ng/mL, respectively. Therefore, the optimal salt concentration was set at 150 mM NaCl in a phosphate buffer solution.

In order to determine the optimal pH of the assay buffer, the IC50 value at a pH of 5.0, 6.0, 6.5, 7.0, 7.5, 8.0, and 9.0 was 8.6, 6.6, 5.5, 5.6, 4.8, 5.2, and 4.8 ng/mL, respectively (Fig. 1B). These results indicated that the evaluation system was more sensitive under neutral to slightly alkaline conditions than under slightly acidic conditions. Therefore, the optimal pH was set to a pH of 7.5.

Under the optimized conditions, the IC50 value was 1.79±0.36 from the competition curve of tetranor-PGDM (Fig. 1C).

Verification of EIA

Tetranor-PGDM was gradually diluted with the assay solution and the limit of detection of tetranor-PGDM was examined. The limit of detection (LOD) was 0.0498 ng/mL, and the range of quantification (ROQ) value was 0.252 to 20.2 ng/mL. The LOD and the ROQ were defined as the 30% RSD concentration and 10% RSD concentration ranges (Fig. 1D).

The inaccuracy of this EIA was evaluated using an assay solution containing tetranor-PGDM at three different concentrations. Intra-and inter-experimental CVs were 3.9% to 6.0% (n=9) and 5.7% to 10.4% (n=9) (Table 4).

[Table 4]

| Concentration (ng/mL) | | | | | Mean Recoveries (%) |
|---|---|---|---|---|---|
| Spiked | Mean | | SD | CV (%) | |
| Within-run (n=9) | | | | | |
| 1.024 | 1.14 | ± | 0.07 | 6.0 | 111.7 |
| 2.56 | 2.29 | ± | 0.11 | 4.8 | 89.4 |
| 6.4 | 6.19 | ± | 0.24 | 3.9 | 96.6 |
| Between-day (n=9) | | | | | |
| 1.024 | 1.10 | ± | 0.11 | 10.4 | 107.6 |
| 2.56 | 2.45 | ± | 0,14 | 5.7 | 95.5 |
| 6.4 | 6.25 | ± | 0.41, | 6.6 | 97.7 |

The recovery of tetranor-PGDM was 89.4% to 111.7%. All recoveries and coefficients of variation of tetranor-PGDM were within the acceptable range.

Cross-reactivity of produced Mab with respect to tetranor-PGDM

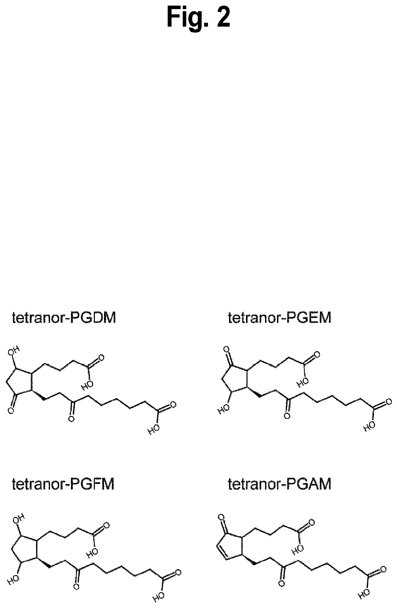

In addition to tetranor-PGDM, other urinary PG metabolites such as tetranor-PGEM and tetranor-PGFM were present in urine (Fig. 2). Next, the cross-reactivity with respect to tetranor-PGEM, tetranor-PGAM, and tetranor-PGFM was evaluated. The cross-reactivity with tetranor-PGEM, tetranor-PGFM, and tetranor-PGAM was 0.00%, 0.00%, and 1.17%, respectively (Table 5).

[Table 5]

| **Lipid** | **Cross-reactivity (%)** |
|---|---|
| **tetranor-PGDM** | **100** |
| **tetranor-PGEM** | **<0.001** |
| **tetranor-PGFM** | **<0.001** |
| **tetranor-PGAM** | **1.17** |

Since no significant cross-reactivity with these metabolites was shown in the developed EIA method, it was suggested that urinary tetranor-PGDM could be measured with sufficient specificity using this EIA method.

Measurement of tetranor-PGDM in artificial urine

A known amount of tetranor-PGDM was added to artificial urine, and recovery of tetranor-PGDM was measured by a competitive EIA method using 60-141Mab.

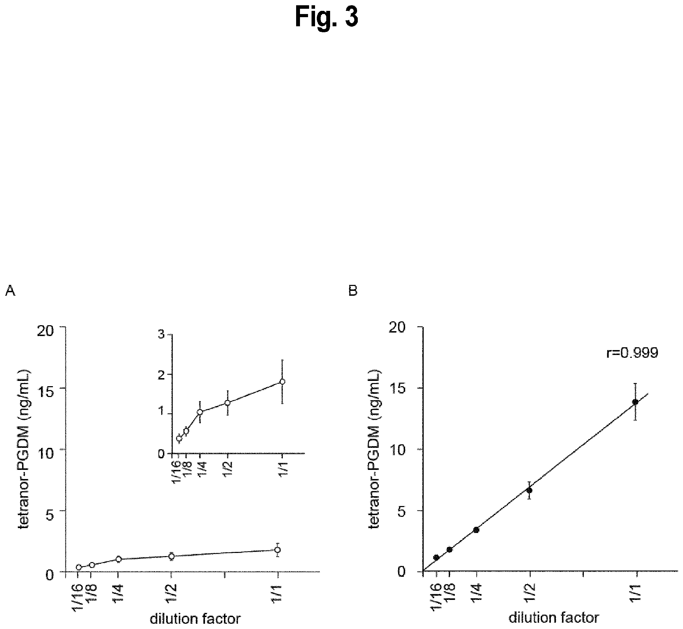

The dilution linearity was an indicator of efficacy of the EIA method using an anti-tetranor-PGDM monoclonal antibody. First, an artificial urine sample containing 20 ng/mL of tetranor-PGDM was gradually diluted and the recovery of tetranor-PGDM according to the antibody was measured. The tetranor-PGDM measured value was not linearly correlated with a dilution factor. In addition, the recovery of tetranor-PGDM was less than 30.2% (Fig. 3A). These results suggested that some urine components may cause a negative interference with the EIA method.

Therefore, the urine sample was subjected to solid phase extraction (SPE) in order to eliminate the negative interference. The recovery of tetranor-PGDM from SPE determined through LC-MS/MS was 77.1% (Table 2). After SPE, an artificial urine sample containing 13.9 ng/mL of tetranor-PGDM was gradually diluted and measured. After SPE, the measured value of the artificial urine showed good linearity with a dilution factor (r=0.999, Fig. 3B).

Tetranor-PGDM with different concentrations was added to artificial urine, and the recovery was determined. The recovery of tetranor-PGDM was in a range of 82.3% to 113.5%. All recoveries of tetranor-PGDM were within the acceptable range (Table 6).

[Table 6]

| | Concentration (ng/mL) | | | Mean Recoveries (%) |
|---|---|---|---|---|
| Sample | Spiked | Theoretical | Measured | |
| AU-1 (n=3) | 0 | - | 0.85 | - |
| | 0.86 | 1.32 | 1.49 | 113.5 |
| | 1,87 | 2.10 | 2.09 | 100.0 |
| | 3.91 | 3.67 | 3.81 | 103.8 |
| | 9.12 | 7.68 | 7.04 | 93.0 |
| AU-2 (n=3) | 0 | - | 5.95 | - |
| | 0.86 | 5.25 | 5.53 | 105.6 |
| | 1.87 | 6.03 | 6.53 | 108.4 |
| | 3.91 | 7.60 | 6.99 | 91.9 |
| | 9.12 | 11 62 | 9.53 | 82.3 |

The concentration of tetranor-PGDM was 0.85 (AU-1) and 5.95 (AU-2).

Conclusion

In this study, for tetranor-PGDM measurement, an anti-tetranor-PGDM monoclonal antibody was prepared and a highly sensitive competitive EIA method was developed. A high average recovery (89.4% to 111.7%) with a coefficient of variation of 3.9% to 10.4% was obtained in the assay solution sample. EIA ROQ for tetranor-PGDM measurement was 0.252 to 196 20.2 ng/mL. The matrix effect of the sample was eliminated by using SPE. A high average recovery (82.3% to 113.5%) was obtained in the urine sample after SPE.

Since antigen-antibody binding was characterized as weak intermolecular binding, a change in either the ionic strength or the pH could influence this interaction. Individual properties of respective urine samples could influence the reliability of analysis using the EIA method [10]. Up to a pH of 9.0, the EIA $IC_{50}$ value did not change significantly. Even if the ionic strength increased to 300 mM, recognition of tetranor-PGDM did not significantly change. These results suggested that a change in either the ionic strength or the pH had had little influence on the EIA method using 60-141 antibodies.

In healthy individuals, the urinary tetranor-PGDM concentration was 1.5±0.3 ng/mg creatinine (Cre) [1]. The urinary Cre concentration was about 1 mg/mL [11]. The inventors previously performed the oral load test for food allergy patients and found that the optimal cutoff value of the urinary tetranor-PGDM was 2.25 ng/mg Cre [3]. The LOQ value was lower than the cutoff value. In other diseases, it has been reported that the urinary tetranor-PGDM concentration in Duchenne muscular dystrophy patients was 9.7 ng/mg Cre [4]. These results indicated that this optimized EIA method was acceptable for detection of urinary tetranor-PGDM in food allergy or Duchenne muscular dystrophy patients.

The urinary tetranor-PGEM and tetranor-PGFM concentrations were 8 to 15 ng/mg Cre and 11 to 59 ng/mL [12, 13]. In this competitive EIA method, the cross-reactivity with other tetranor-PG metabolites was negligible. Mice were immunized using KLH-conjugated tetranor-PGDM prepared by binding KLH to the carboxyl group of tetranor-PGDM via NHS/EDC-mediated esterification. Generally, it was thought that the antibody best recognized the hapten moiety furthest away from the conjugate bond. Therefore, the epitope of the 60-141 antibody was thought to be on the cyclopentane ring of tetranor-PGDM. In addition, the 60-141 antibody did not recognize tetranor-PGEM, tetranor-PGFM, or tetranor-PGAM without the 11-keto group.

This suggested that the epitope of the 60-141 antibody was the 11-keto group of tetranor-PGDM. That is, according to the cross-reactivity test, it was shown that the 60-141 antibody was directed almost exclusively against the tetranor-PGDM structure.

In addition, the 60-141 antibody had a sufficiently high specificity for PGDM compared to other lipids, such as 0.631% for tetranor-PGEM, 3.876% for tetranor-PGAM, and <0.003% for tetranor-PGFM.

In this study, one monoclonal antibody was successfully produced and a highly sensitive competitive EIA method was developed. This EIA method was useful for quantification of tetranor-PGDM in body fluids, evaluation of animal disease models, diagnosis indicators, and treatment monitoring of food allergies and other diseases.

References

1. Song WL, Wang M, Ricciotti E, Fries S, Yu Y, Grosser T, et al. Tetranor PGDM, an abundant urinary metabolite reflects biosynthesis of prostaglandin D2 in mice and humans. J Biol Chem. 2008; 283(2):1179-88. Epub 2007/11/13. doi: 10.1074/jbc.M706839200. PubMed PMID: 17993463.

2. Maeda S, Nakamura T, Harada H, Tachibana Y, Aritake K, Shimosawa T, et al. Prostaglandin D2 metabolite in urine is an index of food allergy. Sci Rep. 2017; 7(1): 17687. Epub 2017/12/17. doi: 10.1038/s41598-017-17798-w. PubMed PMID: 29247205; PubMed Central PMCID: PMCPMC5732293.

3. Inagaki S, Maeda S, Narita M, Nakamura T, Shimosawa T, Murata T, et al. Urinary PGDM, a prostaglandin D2 metabolite, is a novel biomarker for objectively detecting allergic reactions of food allergy. J Allergy Clin Immunol. 2018; 142(5):1634-6.e10. Epub 2018/07/10. doi: 10.1016/j.jaci.2018.06.032. PubMed PMID: 29981807.

4. Takeshita E, Komaki H, Tachimori H, Miyoshi K, Yamamiya I, Shimizu-Motohashi Y, et al. Urinary prostaglandin metabolites as Duchenne muscular dystrophy progression markers. Brain Dev. 2018; 40(10):918-25. Epub 2018/07/15. doi: 10.1016/j.braindev.2018.06.012. PubMed PMID: 30006121.

5. Higashi N, Mita H, Yamaguchi H, Fukutomi Y, Akiyama K, Taniguchi M. Urinary tetranor-PGDM concentrations in aspirin-intolerant asthma and anaphylaxis. J Allergy Clin Immunol. 2012; 129(2):557-9, 9.e1-2. Epub 2011/10/25. doi: 10.1016/j.jaci.2011.09.019. PubMed PMID: 22018904.

6. Harlow E, Lane D. Antibodies: a laboratory manual: Cold Spring Harbor Laboratory; 1988. xiii, 726 p. p.

7. Queffelec AL, Boisde F, Larue JP, Haelters JP, Corbel B, Thouvenot D, et al. Development of an immunoassay (ELISA) for the quantification of thiram in lettuce. J Agric Food Chem. 2001; 49(4):1675-80. Epub 2001/04/20. doi: 10.1021/jf000937j. PubMed PMID: 11308309.

8. Dudley RA, Edwards P, Ekins RP, Finney DJ, McKenzie IG, Raab GM, et al. Guidelines for immunoassay data processing. Clin Chem. 1985; 31(8):1264-71. Epub 1985/08/01. PubMed PMID: 3893796.

9. Hayashi Y, Matsuda R, Maitani T, Imai K, Nishimura W, Ito K, et al. Precision, limit of detection and range of quantitation in competitive ELISA. Anal Chem. 2004; 76(5):1295-301. Epub 2004/02/28. doi: 10.1021/ac0302859. PubMed PMID: 14987084.

10. Schneider D, Gee S, Kreissig S, Harris A, Kramer P, Marco M, et al. New frontiers in agrochemical immunoassay. AOAC International, Washington DC. 1995:103-21.

11. Smith-Palmer T. Separation methods applicable to urinary creatine and creatinine. J Chromatogr B Analyt Technol Biomed Life Sci. 2002; 781(1-2):93-106. Epub 2002/11/27. doi: 10.1016/s1570-0232(02)00617-7. PubMed PMID: 12450655.

12. Granstrom E, Samuelsson B. On the metabolism of prostaglandin F 2 in female subjects. II. Structures of six metabolites. J Biol Chem. 1971; 246(24):7470-85. Epub 1971/12/25. PubMed PMID: 5135312.

13. Zhang Y, Zhang G, Clarke PA, Huang JT, Takahashi E, Muirhead D, et al. Simultaneous and high-throughput quantitation of urinary tetranor PGDM and tetranor PGEM by online SPE-LC-MS/MS as inflammatory biomarkers. J Mass Spectrom. 2011; 46(7):705-11. Epub 2011/06/28. doi: 10.1002/jms.1941. PubMed PMID: 21706677.

**Sequence Listing Free Text**

SEQ ID NO: 1: amino acid sequence of CDRH1: GYT-Xaa1-T-Xaa2-Y-Xaa3, wherein Xaa1 is F or A, Xaa2 is N or R, and Xaa3 is A or W;

SEQ ID NO: 2: amino acid sequence of CDRH2: I-Xaa4-P-Xaa5-Xaa6-Xaa7-Xaa8-T, wherein Xaa4 is N or Y, Xaa5 is F or G, Xaa6 is N or D, Xaa7 is D or G, and Xaa8 is G, N or D;

SEQ ID NO: 3: amino acid sequence of CDRH3: A-Xaa9-Xaa10-Xaa11-Xaa12-Xaa13-Xaa14-Xaa15-Xaa16-Xaa17-Xaa18-Xaa19-Xaa20, wherein Xaa9 is R, S or T, Xaa10 is Y or V, Xaa11 is Y or absent, Xaa12 is Y or absent, Xaa13 is Y or absent, Xaa14 is G or S, Xaa15 is S or R, Xaa16 is K or W, Xaa17 is A or F, Xaa18 is M or F, Xaa19 is D or A, and Xaa20 is Y or V;

SEQ ID NO: 4: amino acid sequence of CDRL1: Xaa21-GAV-Xaa22-Xaa23-S-Xaa24-Xaa25, wherein Xaa21 is T or S, Xaa22 is T or R, Xaa23 is T or I, Xaa24 is N, Y or K, and Xaa25 is Y or H;

SEQ ID NO: 5: amino acid sequence of CDRL3: ALW-Xaa28-SNHW-Xaa29, wherein Xaa28 is Y, F or N, and Xaa29 is V or I;

SEQ ID NO: 6: VH complete base sequence of 31-13 antibody (including signal sequence);

SEQ ID NO: 7: VH complete amino acid sequence of 31-13 antibody (including signal sequence);

SEQ ID NO: 8: VH base sequence of 31-13 antibody (excluding signal sequence);

SEQ ID NO: 9: VH amino acid sequence of 31-13 antibody (excluding signal sequence);

SEQ ID NO: 10: CDRH1 amino acid sequence of 31-13 antibody;

SEQ ID NO: 11: CDRH2 amino acid sequence of 31-13 antibody;

SEQ ID NO: 12: CDRH3 amino acid sequence of 31-13 antibody;

SEQ ID NO: 13: VL complete base sequence of 31-13 antibody (including signal sequence);

SEQ ID NO: 14: VL complete amino acid sequence of 31-13 antibody (including signal sequence);

SEQ ID NO: 15: VL base sequence of 31-13 antibody (excluding signal sequence);

SEQ ID NO: 16: VL amino acid sequence of 31-13 antibody (excluding signal sequence);

SEQ ID NO: 17: CDRL1 amino acid sequence of 31-13 antibody;

SEQ ID NO: 18: CDRL3 amino acid sequence of 31-13 antibody;

SEQ ID NO: 19: VH complete base sequence of 44-63 antibody (including signal sequence);

SEQ ID NO: 20: VH complete amino acid sequence of 44-63 antibody (including signal sequence);

SEQ ID NO: 21: VH base sequence of 44-63 antibody (excluding signal sequence);

SEQ ID NO: 22: VH amino acid sequence of 44-63 antibody (excluding signal sequence);

SEQ ID NO: 23: CDRH1 amino acid sequence of 44-63 antibody;

SEQ ID NO: 24: CDRH2 amino acid sequence of 44-63 antibody;

SEQ ID NO: 25: CDRH3 amino acid sequence of 44-63 antibody;

SEQ ID NO: 26: VL complete base sequence of 44-63 antibody (including signal sequence);

SEQ ID NO: 27: VL complete amino acid sequence of 44-63 antibody (including signal sequence);

SEQ ID NO: 28: VL base sequence of 44-63 antibody (excluding signal sequence);

SEQ ID NO: 29: VL amino acid sequence of 44-63 antibody (excluding signal sequence);

SEQ ID NO: 30: CDRL1 amino acid sequence of 44-63 antibody;

SEQ ID NO: 31: CDRL3 amino acid sequence of 44-63 antibody;

SEQ ID NO: 32: VH complete base sequence of 47-11 antibody (including signal sequence);

SEQ ID NO: 33: VH complete amino acid sequence of 47-11 antibody (including signal sequence);

SEQ ID NO: 34: VH base sequence of 47-11 antibody (excluding signal sequence);

SEQ ID NO: 35: VH amino acid sequence of 47-11 antibody (excluding signal sequence);

SEQ ID NO: 36: CDRH1 amino acid sequence of 47-11 antibody;

SEQ ID NO: 37: CDRH2 amino acid sequence of 47-11 antibody;

SEQ ID NO: 38: CDRH3 amino acid sequence of 47-11 antibody;

SEQ ID NO: 39: VL complete base sequence of 47-11 antibody (including signal sequence);

SEQ ID NO: 40: VL complete amino acid sequence of 47-11 antibody (including signal sequence);

SEQ ID NO: 41: VL base sequence of 47-11 antibody (excluding signal sequence);

SEQ ID NO: 42: VL amino acid sequence of 47-11 antibody (excluding signal sequence);

SEQ ID NO: 43: CDRL1 amino acid sequence of 47-11 antibody;

SEQ ID NO: 44: CDRL3 amino acid sequence of 47-11 antibody;

SEQ ID NO: 45: VH complete base sequence of 50-32 antibody (including signal sequence);

SEQ ID NO: 46: VH complete amino acid sequence of 50-32 antibody (including signal sequence);

SEQ ID NO: 47: VH base sequence of 50-32 antibody (excluding signal sequence);

SEQ ID NO: 48: VH amino acid sequence of 50-32 antibody (excluding signal sequence);

SEQ ID NO: 49: CDRH1 amino acid sequence of 50-32 antibody;

SEQ ID NO: 50: CDRH2 amino acid sequence of 50-32 antibody;

SEQ ID NO: 51: CDRH3 amino acid sequence of 50-32 antibody;

SEQ ID NO: 52: VL complete base sequence of 50-32 antibody (including signal sequence);

SEQ ID NO: 53: VL complete amino acid sequence of 50-32 antibody (including signal sequence);

SEQ ID NO: 54: VL base sequence of 50-32 antibody (excluding signal sequence);

SEQ ID NO: 55: VL amino acid sequence of 50-32 antibody (excluding signal sequence);

SEQ ID NO: 56: CDRL1 amino acid sequence of 50-32 antibody;

SEQ ID NO: 57: CDRL3 amino acid sequence of 50-32 antibody;

SEQ ID NO: 58: VH complete base sequence of 53-113 antibody (including signal sequence);

SEQ ID NO: 59: VH complete amino acid sequence of 53-113 antibody (including signal sequence);

SEQ ID NO: 60: VH base sequence of 53-113 antibody (excluding signal sequence);

SEQ ID NO: 61: VH amino acid sequence of 53-113 antibody (excluding signal sequence);

SEQ ID NO: 62: CDRH1 amino acid sequence of 53-113 antibody;

SEQ ID NO: 63: CDRH2 amino acid sequence of 53-113 antibody;

SEQ ID NO: 64: CDRH3 amino acid sequence of 53-113 antibody;

SEQ ID NO: 65: VL complete base sequence of 53-113 antibody (including signal sequence);

SEQ ID NO: 66: VL complete amino acid sequence of 53-113 antibody (including signal sequence);

SEQ ID NO: 67: VL base sequence of 53-113 antibody (excluding signal sequence);

SEQ ID NO: 68: VL amino acid sequence of 53-113 antibody (excluding signal sequence);

SEQ ID NO: 69: CDRL1 amino acid sequence of 53-113 antibody;

SEQ ID NO: 70: CDRL3 amino acid sequence of 53-113 antibody;

SEQ ID NO: 71: VH complete base sequence of 60-141 antibody (including signal sequence);

SEQ ID NO: 72: VH complete amino acid sequence of 60-141 antibody (including signal sequence);

SEQ ID NO: 73: VH base sequence of 60-141 antibody (excluding signal sequence);

SEQ ID NO: 74: VH amino acid sequence of 60-141 antibody (excluding signal sequence);

SEQ ID NO: 75: CDRH1 amino acid sequence of 60-141 antibody;

SEQ ID NO: 76: CDRH2 amino acid sequence of 60-141 antibody;

SEQ ID NO: 77: CDRH3 amino acid sequence of 60-141 antibody;

SEQ ID NO: 78: VL complete base sequence of 60-141 antibody (including signal sequence);

SEQ ID NO: 79: VL complete amino acid sequence of 60-141 antibody (including signal sequence);

SEQ ID NO: 80: VL base sequence of 60-141 antibody (excluding signal sequence);

SEQ ID NO: 81: VL amino acid sequence of 60-141 antibody (excluding signal sequence);

SEQ ID NO: 82 CDRL1 amino acid sequence of 60-141 antibody; and

SEQ ID NO: 83: CDRL3 amino acid sequence of 60-141 antibody

## Claims

1. A monoclonal antibody or a fragment thereof that specifically binds to tetranor-PGDM.

2. The monoclonal antibody or a fragment thereof according to claim 1, which does not substantially bind to tetranor-PGEM, tetranor-PGFM, or tetranor-PGAM.

3. The monoclonal antibody or a fragment thereof according to claim 1 or 2, which recognizes carbon chains at positions 7 to 11 of tetranor-PGDM.

4. The monoclonal antibody or a fragment thereof according to any one of claims 1 to 3, which comprises at least one or more CDRs selected from the following group consisting of:

(a) CDRH1 consisting of an amino acid sequence as shown in GYT-Xaa1-T-Xaa2-Y-Xaa3 (SEQ ID NO: 1), wherein Xaa1 is F, A or S, Xaa2 is N or R, and Xaa3 is A or W;
(b) CDRH2 consisting of an amino acid sequence as shown in I-Xaa4-P-Xaa5-Xaa6-Xaa7-Xaa8-T (SEQ ID NO: 2), wherein Xaa4 is N or Y, Xaa5 is F or G, Xaa6 is N or D, Xaa7 is D or G, and Xaa8 is G, N or D;
(c) CDRH3 consisting of an amino acid sequence as shown in A-Xaa9-Xaa10-Xaa11-Xaa12-Xaa13-Xaa14-Xaa15-Xaa16-Xaa17-Xaa18-Xaa19-Xaa20 (SEQ ID NO: 3), wherein Xaa9 is R, S or T, Xaa10 is Y or V, Xaa11 is Y or absent, Xaa12 is Y or absent, Xaa13 is Y or absent, Xaa14 is G or S, Xaa15 is S or R, Xaa16 is K or W, Xaa17 is A or F, Xaa18 is M or F, Xaa19 is D or A, and Xaa20 is Y or V;
(d) CDRL1 consisting of an amino acid sequence as shown in Xaa21-GAV-Xaa22-Xaa23-S-Xaa24-Xaa25 (SEQ ID NO: 4), wherein Xaa21 is T or S, Xaa22 is T or R, Xaa23 is T or I, Xaa24 is N, Y or K, and Xaa25 is Y or H;
(e) CDRL2 consisting of an amino acid sequence as shown in Xaa26-T-Xaa27, wherein Xaa26 is G or D, and Xaa27 is N or D; and
(f) CDRL3 consisting of an amino acid sequence as shown in ALW-Xaa28-SNHW-Xaa29 (SEQ ID NO: 5), wherein Xaa28 is Y, F or N, and Xaa29 is V or I;

or comprises at least one or more CDRs consisting of amino acid sequences with deletion, substitution or addition of one or several amino acids in the amino acid sequence of each CDR,
or comprises at least one or more CDRs consisting of amino acid sequences with at least 80% identity to the amino acid sequence of each CDR.

5. The monoclonal antibody or a fragment thereof according to claim 4,
wherein, in CDRH1 of (a), when Xaa2 is N, Xaa1 is F, Xaa3 is A; or when Xaa2 is R, Xaa1 is F, A or S.

6. The monoclonal antibody or a fragment thereof according to claim 4 or 5,
wherein, in CDRH2 of (b), when Xaa4 is N, Xaa5 is F, Xaa6 is N, Xaa7 is D, and Xaa8 is G; when Xaa4 is Y, Xaa5 is G, Xaa6 is D, Xaa7 is G, and Xaa8 is N or D.

7. The monoclonal antibody or a fragment thereof according to any one of claims 4 to 6,
wherein, in CDRH3 of (c), when Xaa10 is Y, Xaa9 is R, Xaa11 is Y, Xaa12 is Y, Xaa13 is Y, Xaa14 is G, Xaa15 is S, Xaa16 is K, Xaa17 is A, Xaa18 is M, Xaa19 is D, and Xaa20 is Y; when Xaa10 is V, Xaa9 is S or T, Xaa11 is absent, Xaa12 is absent, Xaa13 is absent, Xaa14 is S, Xaa15 is R, Xaa16 is W, Xaa17 is F, Xaa18 is F, Xaa19 is A, and Xaa20 is V.

8. The monoclonal antibody or a fragment thereof according to any one of claims 4 to 7,
wherein, in CDRL1 of (d), when Xaa22 and Xaa23 are each T, Xaa21 is T or S, Xaa24 is N, Y or K, and Xaa25 is Y or H; when Xaa22 is R and Xaa23 is I, Xaa21 is T, Xaa24 is N, and Xaa25 is Y.

9. The monoclonal antibody or a fragment thereof according to any one of claims 4 to 8,
wherein, in CDRL2 of (e), when Xaa26 is G, Xaa27 is not D.

10. The monoclonal antibody or a fragment thereof according to any one of claims 4 to 9,
wherein, in CDRL3 of (f), when Xaa28 is Y, Xaa29 is V or I; when Xaa28 is F, Xaa29 is V; or when Xaa28 is N, Xaa29 is V.

11. The monoclonal antibody or a fragment thereof according to any one of claims 1 to 10,
wherein a constant region is derived from a human.

12. The monoclonal antibody or a fragment thereof according to any one of claims 1 to 11, which is human chimerized or humanized.

13. The monoclonal antibody or a fragment thereof according to any one of claims 1 to 12, which is selected from the group consisting of Fab, F(ab')2, Fab', Fv and single chain antibodies.

**14.** A polynucleotide encoding the monoclonal antibody or a fragment thereof according to any one of claims 1 to 13.

**15.** An expression vector comprising the polynucleotide according to claim 14.

**16.** A host cell transfected with the expression vector according to claim 15.

**17.** The host cell according to claim 16, which is a eukaryotic cell.

**18.** A hybridoma that produces the monoclonal antibody according to any one of claims 1 to 13.

**19.** A diagnostic agent for tetranor-PGDM-related disease, comprising the monoclonal antibody or a fragment thereof according to any one of claims 1 to 13.

**20.** The diagnostic agent according to claim 19,
wherein the tetranor-PGDM-related disease is a food allergy, an anaphylactic reaction, muscular dystrophy, or aspirin intolerant asthma.

**21.** A kit comprising the monoclonal antibody or a fragment thereof according to any one of claims 1 to 13 or the diagnostic agent according to claim 19 or 20.

**22.** The kit according to claim 21, further comprising a solid phase carrier,
wherein the monoclonal antibody or a fragment thereof is immobilized on the solid phase carrier.

**23.** A method for detecting tetranor-PGDM using the monoclonal antibody or a fragment thereof according to any one of claims 1 to 13 or the diagnostic agent according to claim 19 or 20.

**24.** A method of assisting in determining whether a subject has a food allergy, comprising:

a step of detecting a urinary tetranor-PGDM level using the monoclonal antibody or a fragment thereof according to any one of claims 1 to 13 or using the diagnostic agent according to claim 19 or 20; and
a step of assisting in determining that, when the urinary tetranor-PGDM level is higher, food allergy symptoms are severe or have become severe, or a risk of developing a food allergy is higher or has become higher.

**25.** The method according to claim 24, further comprising a step of measuring a urinary tetranor-PGEM level.

# Fig. 1

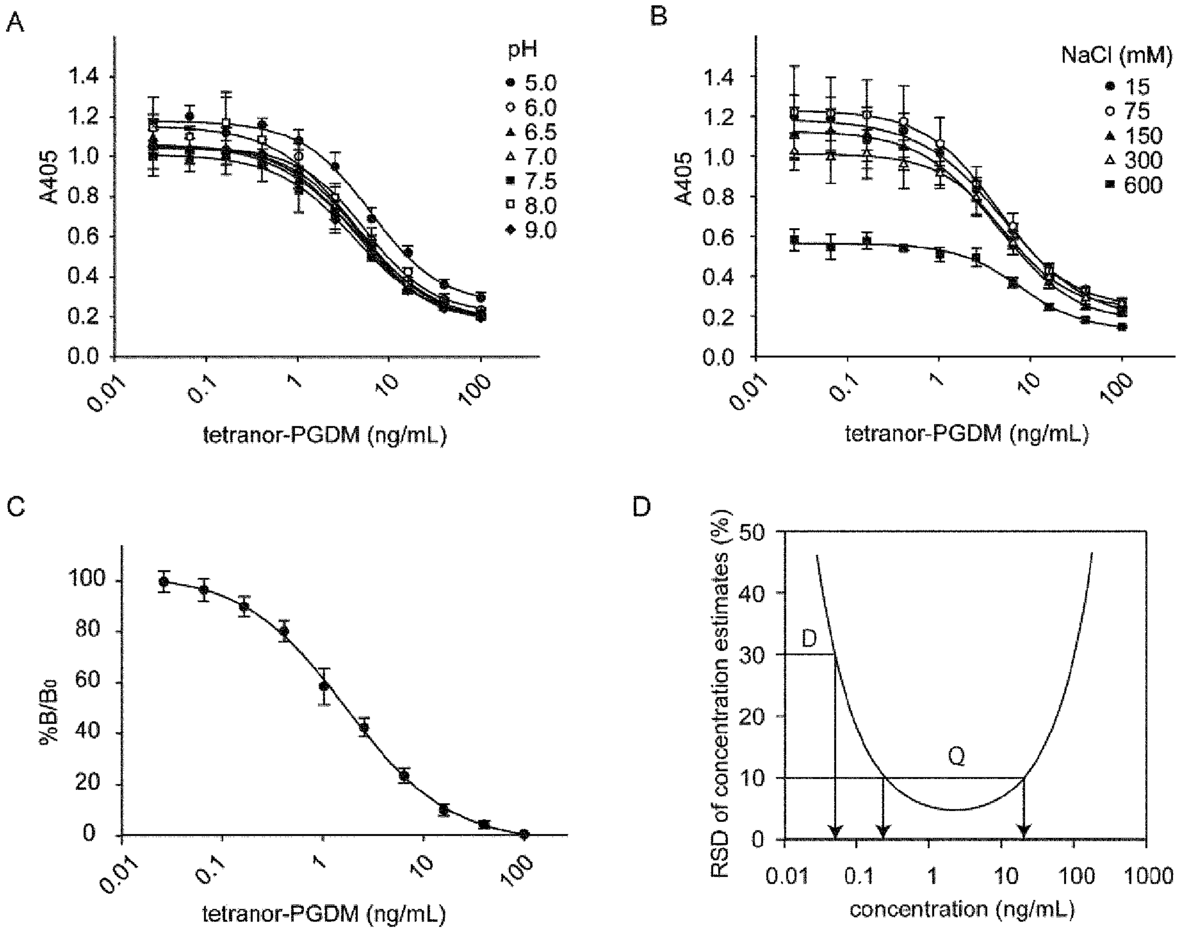
A
pH
5.0
6.0
6.5
7.0
7.5
8.0
9.0
A405
tetranor-PGDM (ng/mL)
B
NaCl (mM)
15
75
150
300
600
A405
tetranor-PGDM (ng/mL)
C
%B/B0
tetranor-PGDM (ng/mL)
D
D
Q
RSD of concentration estimates (%)
concentration (ng/mL)

# Fig. 2

tetranor-PGDM

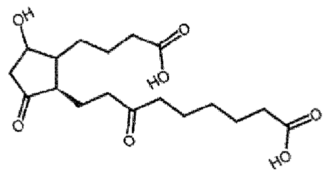

tetranor-PGEM

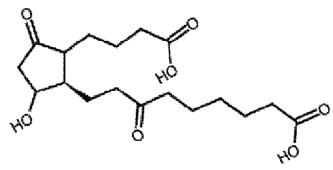

tetranor-PGFM

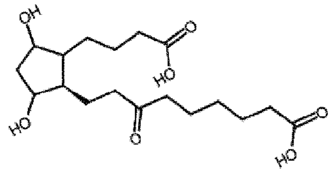

tetranor-PGAM

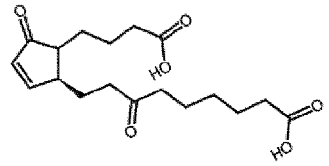

# Fig. 3

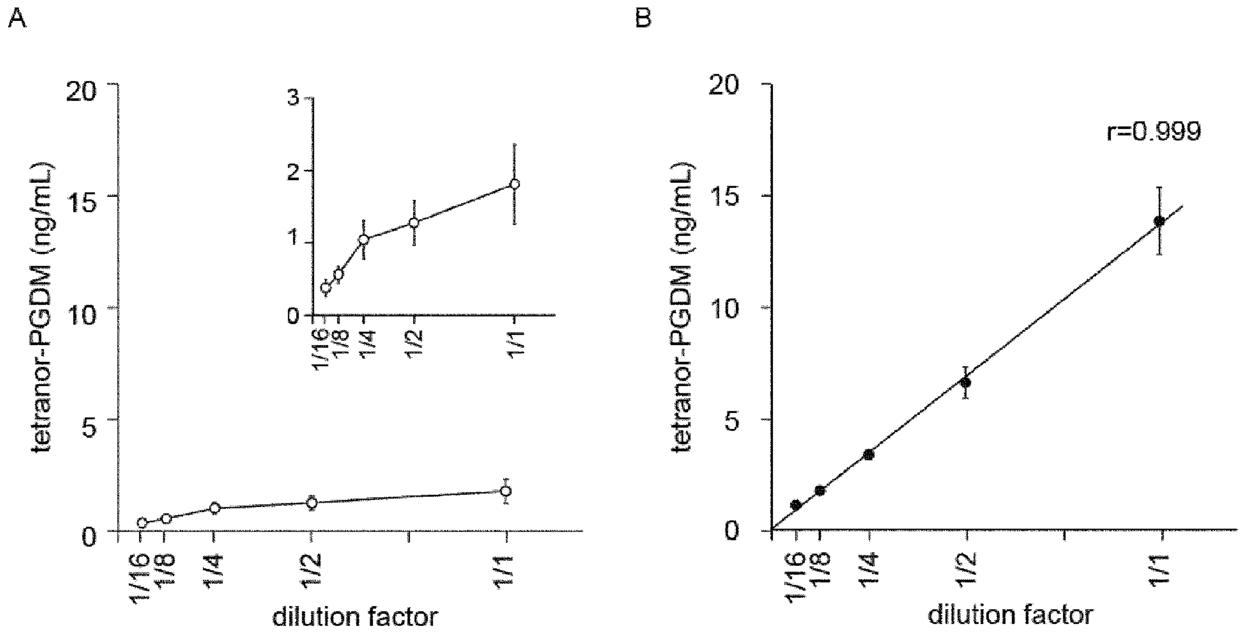
A
tetranor-PGDM (ng/mL)
20
15
10
5
0
3
2
1
0
1/16
1/8
1/4
1/2
1/1
dilution factor
B
r=0.999
tetranor-PGDM (ng/mL)
20
15
10
5
0
1/16
1/8
1/4
1/2
1/1
dilution factor

# Fig. 4

31-13-H

VH COMPLETE BASE SEQUENCE OF ANTIBODY (INCLUDING SIGNAL SEQUENCE)

>31-13-H-Signal plus

ATGGAATGGAGTTGGATATTTCTCTTTCTCCTGTCAGGAACTGCAGGTGTCCACTCTGAGGTCCAGCTGCAGCAGTCTGGACCTGAGCTGGTAAAGCCTGGGGCTTCAGTGAAGATGTCCTGCAAGGC
TTCTGGATACACATTCACTAACTATGCTATGTACTGGGTGAAGCAGAAACCTGGGCAGGGCCTTGAGTGGATTGGATATATTAATCCTTTCAATGACGGTACTAAGAACAATGAGAAGTTCAAAGGCA
AGGCCACACTGACTTCAGACAAATCCTCCAGCACAGCCTACATGGAGCTCAGCAGCCTGACCTCTGAGGACTCTGCGGTCTATTACTGTGCAAGATATTATTACTACGGTAGCAAGGCTATGGACTAC
TGGGGTCAAGGAACCGCAGTCACCGTCTCCTCA

VH COMPLETE AMINO ACID SEQUENCE OF ANTIBODY (INCLUDING SIGNAL SEQUENCE)

>31-13-H-AA-Signal plus

MEWSWIFLFLLSGTAGVHSEVQLQQSGPELVKPGASVKMSCKASGYTFTNYAMYWVKQKPGQGLEWIGYINPFNDGTKNNEKFKGKATLTSDKSSSTAYMELSSLTSEDSAVYYCARYYYYG
SKAMDYWGQGTAVTVSS

VH BASE SEQUENCE OF ANTIBODY (EXCLUDING SIGNAL SEQUENCE)

>31-13-H-Signal minus

GAGGTCCAGCTGCAGCAGTCTGGACCTGAGCTGGTAAAGCCTGGGGCTTCAGTGAAGATGTCCTGCAAGGCTTCTGGATACACATTCACTAACTATGCTATGTACTGGGTGAAGCAGAAACC
TGGGCAGGGCCTTGAGTGGATTGGATATATTAATCCTTTCAATGACGGTACTAAGAACAATGAGAAGTTCAAAGGCAAGGCCACACTGACTTCAGACAAATCCTCCAGCACAGCCTACATGG
AGCTCAGCAGCCTGACCTCTGAGGACTCTGCGGTCTATTACTGTGCAAGATATTATTACTACGGTAGCAAGGCTATGGACTACTGGGGTCAAGGAACCGCAGTCACCGTCTCCTCA

VH AMINO ACID SEQUENCE OF ANTIBODY (EXCLUDING SIGNAL SEQUENCE)

>31-13-H-AA-Signal minus

EVQLQQSGPELVKPGASVKMSCKASGYTFTNYAMYWVKQKPGQGLEWIGYINPFNDGTKNNEKFKGKATLTSDKSSSTAYMELSSLTSEDSAVYYCARYYYYGSKAMDYWGQGTAVTVSS

CDR1 AMINO ACID SEQUENCE OF ANTIBODY VH GYTFTNYA
CDR2 AMINO ACID SEQUENCE OF ANTIBODY VH INPFNDGT
CDR3 AMINO ACID SEQUENCE OF ANTIBODY VH ARYYYYGSKAMDY

# Fig. 5

31-13-L

VL COMPLETE BASE SEQUENCE OF ANTIBODY (INCLUDING SIGNAL SEQUENCE)

>31-13-L-Signal plus

ATGGCCTGGATTTCACTTATACTCTCTCCTGGCTCTCAGCTCAGGGGCCATTTCCCAGGCTGTTGTGACTCAGGAATCTGCACTCACCACATCACCTGGTGAAACAGTCACACTCACTTG
TCGCTCAAGTACTGGGGCTGTTACAACTAGTAACTATGCCAACTGGGTCCAAGAAAAACCAGATCATTTATTCACTGGTCTAATAGGTGGTACCAACAACCGAGCTCCAGGTGTTCCTGCCA
GATTCTCAGGCTCCCTGATTGGAGACAAGGCTGCCCTCACCATCACAGGGGCACAGACTGAGGATGAGGCAATATATTTCTGTGCTCTATGGTACAGCAACCATTGGGTGTTCGGTGGAGGA
ACCAAACTGACTGTCCTA

VL COMPLETE AMINO ACID SEQUENCE OF ANTIBODY (INCLUDING SIGNAL SEQUENCE)

>31-13-L-AA-Signal plus

MAWISLILSLLALSSGAISQAVVTQESALTTSPGETVTLTCRSSTGAVTTSNYANWVQEKPDHLFTGLIGGTNNRAPGVPARFSGSLIGDKAALTITGAQTEDEAIYFCALWYSNHWVFGGG
TKLTVL

VL BASE SEQUENCE OF ANTIBODY (EXCLUDING SIGNAL SEQUENCE)

>31-13-L-Signal minus

CAGGCTGTTGTGACTCAGGAATCTGCACTCACCACATCACCTGGTGAAACAGTCACACTCACTTGTCGCTCAAGTACTGGGGCTGTTACAACTAGTAACTATGCCAACTGGGTCCAAGAAAA
ACCAGATCATTTATTCACTGGTCTAATAGGTGGTACCAACAACCGAGCTCCAGGTGTTCCTGCCAGATTCTCAGGCTCCCTGATTGGAGACAAGGCTGCCCTCACCATCACAGGGGCACAGA
CTGAGGATGAGGCAATATATTTCTGTGCTCTATGGTACAGCAACCATTGGGTGTTCGGTGGAGGAACCAAACTGACTGTCCTA

VL AMINO ACID SEQUENCE OF ANTIBODY (EXCLUDING SIGNAL SEQUENCE)

>31-13-L-AA-Signal minus

QAVVTQESALTTSPGETVTLTCRSSTGAVTTSNYANWVQEKPDHLFTGLIGGTNNRAPGVPARFSGSLIGDKAALTITGAQTEDEAIYFCALWYSNHWVFGGGTKLTVL

CDR1 AMINO ACID SEQUENCE OF ANTIBODY VL TGAVTTSNY
CDR2 AMINO ACID SEQUENCE OF ANTIBODY VL GTN
CDR3 AMINO ACID SEQUENCE OF ANTIBODY VL ALWYSNHWV

# Fig. 6

<u>44-63-H</u>

VH COMPLETE BASE SEQUENCE OF ANTIBODY (INCLUDING SIGNAL SEQUENCE)

>44-63-H-signal plus

ATGGAATGTAACTGGATACTTCCTTTTATTCTGTCAGTAACTTCAGGTGTCTACTCACGGGTTCAGCTCCAGCAGTCTGGGGCTGAGGTGGCTAGACCTGGGGCTTCAGTGGAATTGTCCTG
CAAGTCTTCTGGCTACACCGCAACTCGCTACTGGGTGCAGTGGATAAGACAGAGACCTGGGCAGGGTCTGGAATGGATTGGGGCCATTTATCCTGGAGATGGTAATACTTGGTACACTCAGA
GATTCAAGGACAAGGCCGCATTGACTGTAGATAAAACCTCCAGCACTGCCTACATGCGACTCAGCAGCTTGACATCTGAGGACTCTGCGGTCTTTTACTGTGCAAGCGTCTCCCGGTGGTTC
TTCGCTGTCTGGGGCGCAGGGACCACGGTCACCGTCTCCTCG

VH COMPLETE AMINO ACID SEQUENCE OF ANTIBODY (INCLUDING SIGNAL SEQUENCE)

>44-63-H-AA-signal plus

MECNWILPFILSVTSGVYSRVQLQQSGAEVARPGASVELSCKSSGYTATRYWVQWIRQRPGQGLEWIGAIYPGDGNTWYTQRFKDKAALTVDKTSSTAYMRLSSLTSEDSAVFYCASVSRWF
FAVWGAGTTVIVSS

VH BASE SEQUENCE OF ANTIBODY (EXCLUDING SIGNAL SEQUENCE)

>44-63-H-signal minus

CGGGTTCAGCTCCAGCAGTCTGGGGCTGAGGTGGCTAGACCTGGGGCTTCAGTGGAATTGTCCTGCAAGTCTTCTGGCTACACCGCAACTCGCTACTGGGTGCAGTGGATAAGACAGAGACC
TGGGCAGGGTCTGGAATGGATTGGGGCCATTTATCCTGGAGATGGTAATACTTGGTACACTCAGAGATTCAAGGACAAGGCCGCATTGACTGTAGATAAAACCTCCAGCACTGCCTACATGC
GACTCAGCAGCTTGACATCTGAGGACTCTGCGGTCTTTTACTGTGCAAGCGTCTCCCGGTGGTTCTTCGCTGTCTGGGGCGCAGGGACCACGGTCACCGTCTCCTCG

VH AMINO ACID SEQUENCE OF ANTIBODY (EXCLUDING SIGNAL SEQUENCE)

>44-63-H-AA-signal minus

RVQLQQSGAEVARPGASVELSCKSSGYTATRYWVQWIRQRPGQGLEWIGAIYPGDGNTWYTQRFKDKAALTVDKTSSTAYMRLSSLTSEDSAVFYCASVSRWFFAVWGAGTTVTVSS

CDR1 AMINO ACID SEQUENCE OF ANTIBODY VH GYTATRYW
CDR2 AMINO ACID SEQUENCE OF ANTIBODY VH IYPGDGNT
CDR3 AMINO ACID SEQUENCE OF ANTIBODY VH ASVSRWFFAV

# Fig. 7

44-63-L

VL COMPLETE BASE SEQUENCE OF ANTIBODY (INCLUDING SIGNAL SEQUENCE)

>44-63-L-Signal plus

ATGGCCTGGATTTCACTTATACTCTCTCTCCTGGCTCTCAGCTCAGGGGCCATTTCCCAGGCTATTGTGACTCAGGAATCTGCACTCACCACATCACCTGGTGAAACAGTCACACTCACTTG
TCGCTCAAGTTCTGGGGCTGTTACAACTAGTAACCACGCCAACTGGGTCCAAGAAAAACCAGATCATTTATTCACTGGTCTAATAGGTGATACCAACAACCGAGCTCCAGGTGTTCCTGCCA
GATTCTCAGGCTCCCTGATTGGAGACAAGGCTGCCCTCACCATCACAGGGGCACAGACTGAGGATGAGGCAATATATTTCTGTGCTCTGTGGTTCAGCAACCACTGGGTGTTCGGTGGAGGA
ACCAAACTGACTGTCCTA

VL COMPLETE AMINO ACID SEQUENCE OF ANTIBODY (INCLUDING SIGNAL SEQUENCE)

>44-63-L-AA-Signal plus

MAWISLILSLLALSSGAISQAIVTQESALTTSPGETVTLTCRSSSGAVTTSNHANWVQEKPDHLFTGLIGDTNNRAPGVPARFSGSLIGDKAALTITGAQTEDEAIYFCALWFSNHWVFGGG
TKLTVL

VL BASE SEQUENCE OF ANTIBODY (EXCLUDING SIGNAL SEQUENCE)

>44-63-L-Signal minus

CAGGCTATTGTGACTCAGGAATCTGCACTCACCACATCACCTGGTGAAACAGTCACACTCACTTGTCGCTCAAGTTCTGGGGCTGTTACAACTAGTAACCACGCCAACTGGGTCCAAGAAAA
ACCAGATCATTTATTCACTGGTCTAATAGGTGATACCAACAACCGAGCTCCAGGTGTTCCTGCCAGATTCTCAGGCTCCCTGATTGGAGACAAGGCTGCCCTCACCATCACAGGGGCACAGA
CTGAGGATGAGGCAATATATTTCTGTGCTCTGTGGTTCAGCAACCACTGGGTGTTCGGTGGAGGAACCAAACTGACTGTCCTA

VL AMINO ACID SEQUENCE OF ANTIBODY (EXCLUDING SIGNAL SEQUENCE)

>44-63-L-AA-Signal minus

QAIVTQESALTTSPGETVTLTCRSSSGAVTTSNHANWVQEKPDHLFTGLIGDTNNRAPGVPARFSGSLIGDKAALTITGAQTEDEAIYFCALWFSNHWVFGGGTKLTVL

CDR1 AMINO ACID SEQUENCE OF ANTIBODY VL SGAVTTSNH
CDR2 AMINO ACID SEQUENCE OF ANTIBODY VL DTN
CDR3 AMINO ACID SEQUENCE OF ANTIBODY VL ALWFSNHWV

# Fig. 8

47-11-H

VH COMPLETE BASE SEQUENCE OF ANTIBODY (INCLUDING SIGNAL SEQUENCE)

>47-11-H-Signal plus

ATGGAATGTAACTGGATACTTCCTTTTATTCTGTCAGTAACTTCAGGTGTCTACTCACAAATTCAGCTCCAGCAGTCTGGGGCTGAGGTGGCAAGACCTGGGACTTCAATGAGATTGTCCTG
CAAGGCTTCTGGCTACACCTTTACTCGCTACTGGATGCAGTGGGTAAAACAGAGGCCTGGACAGGGTCTGGAATGGATTGGGGCTATTTATCCTGGAGATGGTAATACTTGGTATACTCAGA
AATTCAAGGACAAGGCCACATTGACTATAGATAAAACCTCCGGCACAGCCTACATTCAACTCATCAGCTTGGCATCTGAGGACTCTGCGGTCTATTACTGTGCAACAGTCTCCCGGTGGTTT
TTCGCTGTCTGGGGCGCAGGGACAACGGTCACCGTCTCCTCA

VH COMPLETE AMINO ACID SEQUENCE OF ANTIBODY (INCLUDING SIGNAL SEQUENCE)

>47-11-H-AA-Signal plus

MECNWILPFILSVTSGVYSQIQLQQSGAEVARPGTSMRLSCKASGYTFTRYWMQWVKQRPGQGLEWIGAIYPGDGNTWYTQKFKDKATLTIDKTSGTAYIQLISLASEDSAVYYCATVSRWF
FAVWGAGTTVTVSS

VH BASE SEQUENCE OF ANTIBODY (EXCLUDING SIGNAL SEQUENCE)

>47-11-H-Signal minus

CAAATTCAGCTCCAGCAGTCTGGGGCTGAGGTGGCAAGACCTGGGACTTCAATGAGATTGTCCTGCAAGGCTTCTGGCTACACCTTTACTCGCTACTGGATGCAGTGGGTAAAACAGAGGCC
TGGACAGGGTCTGGAATGGATTGGGGCTATTTATCCTGGAGATGGTAATACTTGGTATACTCAGAAATTCAAGGACAAGGCCACATTGACTATAGATAAAACCTCCGGCACAGCCTACATTC
AACTCATCAGCTTGGCATCTGAGGACTCTGCGGTCTATTACTGTGCAACAGTCTCCCGGTGGTTTTTCGCTGTCTGGGGCGCAGGGACAACGGTCACCGTCTCCTCA

VH AMINO ACID SEQUENCE OF ANTIBODY (EXCLUDING SIGNAL SEQUENCE)

>47-11-H-AA-Signal minus

QIQLQQSGAEVARPGTSMRLSCKASGYTFTRYWMQWVKQRPGQGLEWIGAIYPGDGNTWYTQKFKDKATLTIDKTSGTAYIQLISLASEDSAVYYCATVSRWFFAVWGAGTTVTVSS

CDR1 AMINO ACID SEQUENCE OF ANTIBODY VH GYTFTRYW
CDR2 AMINO ACID SEQUENCE OF ANTIBODY VH IYPGDGNT
CDR3 AMINO ACID SEQUENCE OF ANTIBODY VH ATVSRWFFAV

# Fig. 9

47-11-L

VL COMPLETE BASE SEQUENCE OF ANTIBODY (INCLUDING SIGNAL SEQUENCE)

>47-11-L-Signal plus

ATGGCCTGGATTTCACTTATACTCTCTCTCCTGGCTCTCAGCTCAGGGGCCTTTTCCCAGGCTGTTGTGACTCAGGAATCTGCACTCACCACATCACCTGGTGAAACAGTCACACTCACTTG
TCGCTCAAGTACTGGGGCTGTTACAACTAGTTATCATGCCAACTGGGTCCAAGAAAAACCAGATCATTTATTCACTGGTCTAATTGGTGATACCGACAACCGAGCTCCAGGTATTCCTGCCA
GGTTCTCAGGCTCCCTGATTGGAGACAAGGCTGCCCTCACCATCACAGGGGCTCAGCCTGAGGATGAGGCAATCTATTTCTGTGCTCTATGGTACAGCAACCACTGGATCTTCGGTGGAGGA
ACCAAACTGACTGTCCTG

VL COMPLETE AMINO ACID SEQUENCE OF ANTIBODY (INCLUDING SIGNAL SEQUENCE)

>47-11-L-AA-Signal plus

MAWISLILSLLALSSGAFSQAVVTQESALTTSPGETVTLTCRSSTGAVTTSYHANWVQEKPDHLFTGLIGDTDNRAPGIPARFSGSLIGDKAALTITGAQPEDEAIYFCALWYSNHWIFGGG
TKLTVL

VL BASE SEQUENCE OF ANTIBODY (EXCLUDING SIGNAL SEQUENCE)

>47-11-L-Signal minus

CAGGCTGTTGTGACTCAGGAATCTGCACTCACCACATCACCTGGTGAAACAGTCACACTCACTTGTCGCTCAAGTACTGGGGCTGTTACAACTAGTTATCATGCCAACTGGGTCCAAGAAAA
ACCAGATCATTTATTCACTGGTCTAATTGGTGATACCGACAACCGAGCTCCAGGTATTCCTGCCAGGTTCTCAGGCTCCCTGATTGGAGACAAGGCTGCCCTCACCATCACAGGGGCTCAGC
CTGAGGATGAGGCAATCTATTTCTGTGCTCTATGGTACAGCAACCACTGGATCTTCGGTGGAGGAACCAAACTGACTGTCCTG

VL AMINO ACID SEQUENCE OF ANTIBODY (EXCLUDING SIGNAL SEQUENCE)

>47-11-L-AA-Signal minus

QAVVTQESALTTSPGETVTLTCRSSTGAVTTSYHANWVQEKPDHLFTGLIGDTDNRAPGIPARFSGSLIGDKAALTITGAQPEDEAIYFCALWYSNHWIFGGGTKLTVL

CDR1 AMINO ACID SEQUENCE OF ANTIBODY VL TGAVTTSYH
CDR2 AMINO ACID SEQUENCE OF ANTIBODY VL DTD
CDR3 AMINO ACID SEQUENCE OF ANTIBODY VL ALWYSNHWI

# Fig. 10

50-32-H

VH COMPLETE BASE SEQUENCE OF ANTIBODY (INCLUDING SIGNAL SEQUENCE)

>50-32-H-Signal plus

ATGGAATGTAACTGGATACTTCCTTTTATTCTGTCAGTAACTTCAGGTGTCTACTCACAGGTTCAGCTCCAGCAGTCTGGGGCTGAGGTGGCAAGACCTGGGGCTTCAGTGAAATTGTCCTG
CAAGGCTTCTGGCTACACCTTTACTCGCTACTGGATGCAGTGGATAAAACAGAGGCCTGGACAGGGTCTGGAATGGATTGGGGCTATTTATCCTGGAGATGGTGATACTTGGTATACTCAGA
AATTCAAGGACAAGGCCACGTTGACTATAGATAAAACCTCCGGCACCGCCTACATTCAACTCATCAGCTTGGCATCTGAGGACTCTGCGGTCTATTACTGTGCATCAGTCTCCCGGTGGTTC
TTCGCTGTCTGGGGCGCAGGGACCACGGTCACCGTCTCCTCA

VH COMPLETE AMINO ACID SEQUENCE OF ANTIBODY (INCLUDING SIGNAL SEQUENCE)

>50-32-H-AA-Signal plus

MECNWILPFILSVTSGVYSQVQLQQSGAEVARPGASVKLSCKASGYTFTRYWMQWIKQRPGQGLEWIGAIYPGDGDTWYTQKFKDKATLTIDKTSGTAYIQLISLASEDSAVYYCASVSRWF
FAVWGAGTTVTVSS

VH BASE SEQUENCE OF ANTIBODY (EXCLUDING SIGNAL SEQUENCE)

>50-32-H-Signal minus

CAGGTTCAGCTCCAGCAGTCTGGGGCTGAGGTGGCAAGACCTGGGGCTTCAGTGAAATTGTCCTGCAAGGCTTCTGGCTACACCTTTACTCGCTACTGGATGCAGTGGATAAAACAGAGGCC
TGGACAGGGTCTGGAATGGATTGGGGCTATTTATCCTGGAGATGGTGATACTTGGTATACTCAGAAATTCAAGGACAAGGCCACGTTGACTATAGATAAAACCTCCGGCACCGCCTACATTC
AACTCATCAGCTTGGCATCTGAGGACTCTGCGGTCTATTACTGTGCATCAGTCTCCCGGTGGTTCTTCGCTGTCTGGGGCGCAGGGACCACGGTCACCGTCTCCTCA

VH AMINO ACID SEQUENCE OF ANTIBODY (EXCLUDING SIGNAL SEQUENCE)

>50-32-H-AA-Signal minus

QVQLQQSGAEVARPGASVKLSCKASGYTFTRYWMQWIKQRPGQGLEWIGAIYPGDGDTWYTQKFKDKATLTIDKTSGTAYIQLISLASEDSAVYYCASVSRWFFAVWGAGTTVTVSS

CDR1 AMINO ACID SEQUENCE OF ANTIBODY VH GYTFTRYW
CDR2 AMINO ACID SEQUENCE OF ANTIBODY VH IYPGDGDT
CDR3 AMINO ACID SEQUENCE OF ANTIBODY VH ASVSRWFFAV

# Fig. 11

50-32-L

VL COMPLETE BASE SEQUENCE OF ANTIBODY (INCLUDING SIGNAL SEQUENCE)

>50-32-L-Signal plus

ATGGCCTGGATTTCACTTATACTCTCTCTCCTGGCTCTCAGCTCAGGGGCCATTTCCCAGGCTGTTGTGACTCAGGAATCTGCACTCACCACATCACCTGGTGAAACAGTCACACTCACTTG
TCGCTCAAGTACTGGGGCTGTTACAACTAGTAATCATGCCAACTGGGTCCAAGAAAAACCAGATCCTTTATTCACTGGTCTATTAGGTGATACCAACAACCGAGCTCCAGGTGTTCCTGCCA
GATTCTCAGGCTCCCTGATTGGAGACAAGGCTGCCCTCACCATCACAGGGGCACAGCCTGAGGATGAGGCAATATATTTCTGTGCTCTATGGTACAGTAACCACTGGGTGTTCGGTGGAGGA
ACCAAACTGATTGTCCTG

VL COMPLETE AMINO ACID SEQUENCE OF ANTIBODY (INCLUDING SIGNAL SEQUENCE)

>50-32-L-AA-Signal plus

MAWISLILSLLALSSGAISQAVVTQESALTTSPGETVTLTCRSSTGAVTTSNHANWVQEKPDPLFTGLLGDTNNRAPGVPARFSGSLIGDKAALTITGAQPEDEAIYFCALWYSNHWVFGGG
TKLIVL

VL BASE SEQUENCE OF ANTIBODY (EXCLUDING SIGNAL SEQUENCE)

>50-32-L-Signal minus

CAGGCTGTTGTGACTCAGGAATCTGCACTCACCACATCACCTGGTGAAACAGTCACACTCACTTGTCGCTCAAGTACTGGGGCTGTTACAACTAGTAATCATGCCAACTGGGTCCAAGAAAA
ACCAGATCCTTTATTCACTGGTCTATTAGGTGATACCAACAACCGAGCTCCAGGTGTTCCTGCCAGATTCTCAGGCTCCCTGATTGGAGACAAGGCTGCCCTCACCATCACAGGGGCACAGC
CTGAGGATGAGGCAATATATTTCTGTGCTCTATGGTACAGTAACCACTGGGTGTTCGGTGGAGGAACCAAACTGATTGTCCTG

VL AMINO ACID SEQUENCE OF ANTIBODY (EXCLUDING SIGNAL SEQUENCE)

>50-32-L-AA-Signal minus

QAVVTQESALTTSPGETVTLTCRSSTGAVTTSNHANWVQEKPDPLFTGLLGDTNNRAPGVPARFSGSLIGDKAALTITGAQPEDEAIYFCALWYSNHWVFGGGTKLIVL

CDR1 AMINO ACID SEQUENCE OF ANTIBODY VL TGAVTTSNH
CDR2 AMINO ACID SEQUENCE OF ANTIBODY VL DTN
CDR3 AMINO ACID SEQUENCE OF ANTIBODY VL ALWYSNHWV

# Fig. 12

53-113-H

VH COMPLETE BASE SEQUENCE OF ANTIBODY (INCLUDING SIGNAL SEQUENCE)

>53-113-H-Signal plus

ATGGAATGTAACTGGATACTTCCTTTTATTCTGTCAGTAACTTCAGGTGTCTACTCACAGTTTCAGCTCCAGCAGTCTGGGGCTGAGGTGGCTAGACCTGGGTCTTCAGTGAAATTGTCCTG
CAGGGCTTCTGGCTACACCTCAACTCGCTACTGGGTGCAGTGGGTAAAACAGAGACCTGGGCAGGGTCTGGAATGGATTGGGGCTATTTATCCTGGAGATGGTGATACTTGGTACACTCAGA
GATTCAAGGACAAGGCCACATTGACTGCAGATAAAACCTCCAGCACTGCCTACATGCAACTCAGCAGCTTGGCATCTGAGGACTCTGCGGTCTATTACTGTGCAAGCGTCTCCCGGTGGTTC
TTCGCTGTCTGGGGCGCAGGGACCACGGTCACCGTCTCCTCA

VH COMPLETE AMINO ACID SEQUENCE OF ANTIBODY (INCLUDING SIGNAL SEQUENCE)

>53-113-H-AA-Signal plus

MECNWILPFILSVTSGVYSQFQLQQSGAEVARPGSSVKLSCRASGYTSTRYWVQWVKQRPGQGLEWIGAIYPGDGDTWYTQRFKDKATLTADKTSSTAYMQLSSLASEDSAVYYCASVSRWF
FAVWGAGTTVTVSS

VH BASE SEQUENCE OF ANTIBODY (EXCLUDING SIGNAL SEQUENCE)

>53-113-H-Signal minus

CAGTTTCAGCTCCAGCAGTCTGGGGCTGAGGTGGCTAGACCTGGGTCTTCAGTGAAATTGTCCTGCAGGGCTTCTGGCTACACCTCAACTCGCTACTGGGTGCAGTGGGTAAAACAGAGACC
TGGGCAGGGTCTGGAATGGATTGGGGCTATTTATCCTGGAGATGGTGATACTTGGTACACTCAGAGATTCAAGGACAAGGCCACATTGACTGCAGATAAAACCTCCAGCACTGCCTACATGC
AACTCAGCAGCTTGGCATCTGAGGACTCTGCGGTCTATTACTGTGCAAGCGTCTCCCGGTGGTTCTTCGCTGTCTGGGGCGCAGGGACCACGGTCACCGTCTCCTCA

VH AMINO ACID SEQUENCE OF ANTIBODY (EXCLUDING SIGNAL SEQUENCE)

>53-113-H-AA-Signal minus

QFQLQQSGAEVARPGSSVKLSCRASGYTSTRYWVQWVKQRPGQGLEWIGAIYPGDGDTWYTQRFKDKATLTADKTSSTAYMQLSSLASEDSAVYYCASVSRWFFAVWGAGTTVTVSS

CDR1 AMINO ACID SEQUENCE OF ANTIBODY VH GYTSTRYW
CDR2 AMINO ACID SEQUENCE OF ANTIBODY VH IYPGDGDI
CDR3 AMINO ACID SEQUENCE OF ANTIBODY VH ASVSRWFFAV

# Fig. 13

53-113-L

VL COMPLETE BASE SEQUENCE OF ANTIBODY (INCLUDING SIGNAL SEQUENCE)

>53-113-L-Signal plus

ATGGCCTGGATTTCACTTATACTCTCTCTCCTGGCTCTCAGCTCAGGGGCCATTTCCCAGGCTATTGTGACTCAGGAATCTGCACTCACCACATCACCTGGTGAAACAGTCACACTCACTTG
TCGCTCAAGTACTGGGGCTGTTACAACTAGTAAATATGCCAACTGGGTCCAAGAAAAACCAGATCATTTATTCACTGGTCTAATAGGTGGTACCAACAACCGAGCTCCAGGTGTTCCTGCCA
GATTCTCAGGCTCCCTGATTGGAGACAAGGCTGCCCTCACCATCACAGGGGCACAGACTGAGGATGAGGCAATATATTTCTGTGCTCTATGGTACAGCAACCACTGGGTGTTCGGTGGAGGA
ACCAAACTGACTGTCCTA

VL COMPLETE AMINO ACID SEQUENCE OF ANTIBODY (INCLUDING SIGNAL SEQUENCE)

>53-113-L-AA-Signal plus

MAWISLILSLLALSSGAISQAIVTQESALTTSPGETVTLTCRSSTGAVTTSKYANWVQEKPDHLFTGLIGGTNNRAPGVPARFSGSLIGDKAALTITGAQTEDEAIYFCALWYSNHWVFGGG
TKLTVL

VL BASE SEQUENCE OF ANTIBODY (EXCLUDING SIGNAL SEQUENCE)

>53-113-L-Signal minus

CAGGCTATTGTGACTCAGGAATCTGCACTCACCACATCACCTGGTGAAACAGTCACACTCACTTGTCGCTCAAGTACTGGGGCTGTTACAACTAGTAAATATGCCAACTGGGTCCAAGAAAA
ACCAGATCATTTATTCACTGGTCTAATAGGTGGTACCAACAACCGAGCTCCAGGTGTTCCTGCCAGATTCTCAGGCTCCCTGATTGGAGACAAGGCTGCCCTCACCATCACAGGGGCACAGA
CTGAGGATGAGGCAATATATTTCTGTGCTCTATGGTACAGCAACCACTGGGTGTTCGGTGGAGGAACCAAACTGACTGTCCTA

VL AMINO ACID SEQUENCE OF ANTIBODY (EXCLUDING SIGNAL SEQUENCE)

>53-113-L-AA-Signal minus

QAIVTQESALTTSPGETVTLTCRSSTGAVTTSKYANWVQEKPDHLFTGLIGGTNNRAPGVPARFSGSLIGDKAALTITGAQTEDEAIYFCALWYSNHWVFGGGTKLTVL

CDR1 AMINO ACID SEQUENCE OF ANTIBODY VL TGAVTTSKY
CDR2 AMINO ACID SEQUENCE OF ANTIBODY VL GTN
CDR3 AMINO ACID SEQUENCE OF ANTIBODY VL ALWYSNHWV

# Fig. 14

<u>60-141-H</u>

VH COMPLETE BASE SEQUENCE OF ANTIBODY (INCLUDING SIGNAL SEQUENCE)

>60-141-H-Signal plus

ATGGAATGGAGTTGGATATTTCTCTTTCTCCTGTCAGGAACTGCAGGTGTCCACTCTGAGGTCCAGCTGCAGCAGTCTGGACCTGAGCTGGTAAAGCCTGGGGCTTCAGTGAAGATGTCCTG
CAAGGCTTCTGGATACACATTCACTAACTATGCTATGTACTGGGTGAAGCAGAAACCTGGGCAGGGCCTTGAGTGGATTGGATATATTAATCCTTTCAATGACGGTACTAAGAACAATGAGA
AGTTCAAAGGGAAGGCCACACTGACTTCAGACAAATCCTCCAGCACAGCCTACATGGAGCTCAGCAGCCTGACCTCTGAGGACTCTGCGGTCTATTACTGTGCAAGATATTATTACTACGGT
AGCAAGGCTATGGACTACTGGGGTCAAGGAACCGCAGTCACCGTCTCCTCA

VH COMPLETE AMINO ACID SEQUENCE OF ANTIBODY (INCLUDING SIGNAL SEQUENCE)

>60-141-H-AA-Signal plus

MEWSWIFLFLLSGTAGVHSEVQLQQSGPELVKPGASVKMSCKASGYTFTNYAMYWVKQKPGQGLEWIGYINPFNDGTKNNEKFKGKATLTSDKSSSTAYMELSSLTSEDSAVYYCARYYYYG
SKAMDYWGQGTAVTVSS

VH BASE SEQUENCE OF ANTIBODY (EXCLUDING SIGNAL SEQUENCE)

>60-141-H-Signal minus

GAGGTCCAGCTGCAGCAGTCTGGACCTGAGCTGGTAAAGCCTGGGGCTTCAGTGAAGATGTCCTGCAAGGCTTCTGGATACACATTCACTAACTATGCTATGTACTGGGTGAAGCAGAAACC
TGGGCAGGGCCTTGAGTGGATTGGATATATTAATCCTTTCAATGACGGTACTAAGAACAATGAGAAGTTCAAAGGGAAGGCCACACTGACTTCAGACAAATCCTCCAGCACAGCCTACATGG
AGCTCAGCAGCCTGACCTCTGAGGACTCTGCGGTCTATTACTGTGCAAGATATTATTACTACGGTAGCAAGGCTATGGACTACTGGGGTCAAGGAACCGCAGTCACCGTCTCCTCA

VH AMINO ACID SEQUENCE OF ANTIBODY (EXCLUDING SIGNAL SEQUENCE)

>60-141-H-AA-Signal minus

EVQLQQSGPELVKPGASVKMSCKASGYTFTNYAMYWVKQKPGQGLEWIGYINPFNDGTKNNEKFKGKATLTSDKSSSTAYMELSSLTSEDSAVYYCARYYYYGSKAMDYWGQGTAVTVSS

CDR1 AMINO ACID SEQUENCE OF ANTIBODY VH GYTFTNYA
CDR2 AMINO ACID SEQUENCE OF ANTIBODY VH INPFNDGT
CDR3 AMINO ACID SEQUENCE OF ANTIBODY VH ARYYYYGSKAMDY

# Fig. 15

60-141-L

VL COMPLETE BASE SEQUENCE OF ANTIBODY (INCLUDING SIGNAL SEQUENCE)

>60-141-L-Signal plus Signal plus

ATGGCCTGGATTTCACTTATACTCTCTCTCCTGGCTCTCAGCTCAGGGGCCATTTCCCAGGCTGTTGTGACTCAGGAATCTGCACTCACCACATCACCTGGTGAAACAGTCACACTCACTTG
TCGCTCAAATACTGGGGCTGTTAGAATTAGTAACTATGCCAACTGGGTCCGAGAAAAACCAGATCATTTATTCACTGGTCTAATAGGTGGTACCAACAACCGAGCTCCAGGTGTTCCTGCCA
GATTCTCAGGCTCCCTGATTGGAGACAAGGCTGCCCTCACCATCACAGGGGCACAGACTGATGATGAGGCAATATATTTCTGTGCTCTATGGAACAGCAACCATTGGGTGTTCGGTGGAGGA
ACCAAACTGACTGTCCTA

VL COMPLETE AMINO ACID SEQUENCE OF ANTIBODY (INCLUDING SIGNAL SEQUENCE)

>60-141-L-AA-Signal plus

MAWISLILSLLALSSGAISQAVVTQESALTTSPGETVTLTCRSNTGAVRISNYANWVREKPDHLFTGLIGGTNNRAPGVPARFSGSLIGDKAALTITGAQTDDEAIYFCALWNSNHWVFGGG
TKLTVL

VL BASE SEQUENCE OF ANTIBODY (EXCLUDING SIGNAL SEQUENCE)

>60-141-L-Signal plus Signal minus

CAGGCTGTTGTGACTCAGGAATCTGCACTCACCACATCACCTGGTGAAACAGTCACACTCACTTGTCGCTCAAATACTGGGGCTGTTAGAATTAGTAACTATGCCAACTGGGTCCGAGAAAA
ACCAGATCATTTATTCACTGGTCTAATAGGTGGTACCAACAACCGAGCTCCAGGTGTTCCTGCCAGATTCTCAGGCTCCCTGATTGGAGACAAGGCTGCCCTCACCATCACAGGGGCACAGA
CTGATGATGAGGCAATATATTTCTGTGCTCTATGGAACAGCAACCATTGGGTGTTCGGTGGAGGAACCAAACTGACTGTCCTA

VL AMINO ACID SEQUENCE OF ANTIBODY (EXCLUDING SIGNAL SEQUENCE)

>60-141-L-AA-Signal plus Signal minus

QAVVTQESALTTSPGETVTLTCRSNTGAVRISNYANWVREKPDHLFTGLIGGTNNRAPGVPARFSGSLIGDKAALTITGAQTDDEAIYFCALWNSNHWVFGGGTKLTVL

CDR1 AMINO ACID SEQUENCE OF ANTIBODY VL TGAVRISNY
CDR2 AMINO ACID SEQUENCE OF ANTIBODY VL GTN
CDR3 AMINO ACID SEQUENCE OF ANTIBODY VL ALWNSNHWV

Fig. 16

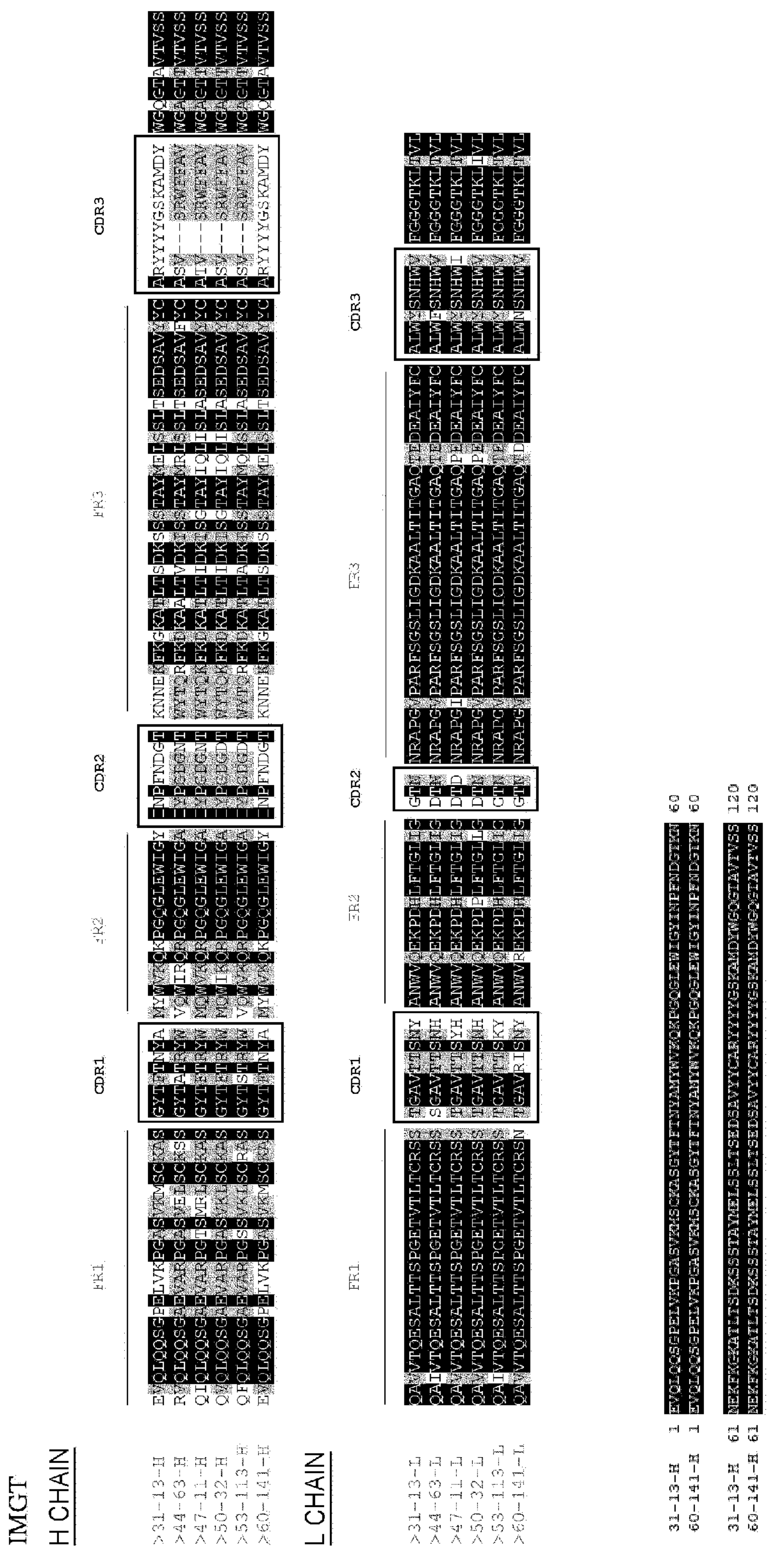
IMGT
H CHAIN
L CHAIN
FR1
CDR1
FR2
CDR2
FR3
CDR3
>31-13-H
>44-63-H
>47-11-H
>50-32-H
>53-113-H
>60-141-H
>31-13-L
>44-63-L
>47-11-L
>50-32-L
>53-113-L
>60-141-L

**INTERNATIONAL SEARCH REPORT**

International application No.
**PCT/JP2022/006890**

**A. CLASSIFICATION OF SUBJECT MATTER**

***C12N 15/13***(2006.01)i; ***A61K 39/395***(2006.01)i; ***A61P 37/08***(2006.01)i; ***C07K 16/18***(2006.01)i; ***C07K 16/44***(2006.01)i; ***C07K 17/00***(2006.01)i; ***C12N 1/15***(2006.01)i; ***C12N 1/19***(2006.01)i; ***C12N 5/10***(2006.01)i; ***C12N 5/12***(2006.01)i; ***G01N 33/50***(2006.01)i; ***G01N 33/53***(2006.01)i

FI: C12N15/13; C07K16/18; C12N1/15; C12N1/19; C12N5/10; C12N5/12; G01N33/53 S; G01N33/50 Z; C07K17/00; A61P37/08; A61K39/395 N; C07K16/44 ZNA

According to International Patent Classification (IPC) or to both national classification and IPC

**B. FIELDS SEARCHED**

Minimum documentation searched (classification system followed by classification symbols)

C12N15/13; A61K39/395; A61P37/08; C07K16/18; C07K16/44; C07K17/00; C12N1/15; C12N1/19; C12N5/10; C12N5/12; G01N33/50; G01N33/53

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2010/104025 A1 (OSAKA BIOSCIENCE INSTITUTE) 16 September 2010 (2010-09-16) claims, paragraphs [0033]-[0035] | 1-25 |
| A | WO 2009/043015 A1 (THE TRUSTEES OF THE UNIVERSITY OF PENNSYLVANIA) 02 April 2009 (2009-04-02) page 10, paragraph 6 to page 11, paragraph 1 | 1-25 |
| A | WO 2016/021704 A1 (THE UNIVERSITY OF TOKYO) 11 February 2016 (2016-02-11) claims, paragraph [0028] | 1-25 |
| A | US 2012/0021437 A1 (CAYMAN CHEMICAL CO., INC.) 26 January 2012 (2012-01-26) claims, example 1 | 1-25 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 April 2022** | **19 April 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/006890**

**C. DOCUMENTS CONSIDERED TO BE RELEVANT**

| Category* | Citation of document, with indication, where appropriate, of the relevant passages | Relevant to claim No. |
|---|---|---|
| P, X | NAGATA, Nanae et al. Development of Monoclonal Antibody-Based EIA for Tetranor-PGDM which Reflects PGD2 Production in the Body. Journal of Immunology Research, 27 April 2021, vol. 2021, Article ID 5591115 abstract | 1-25 |

Form PCT/ISA/210 (second sheet) (January 2015)

**INTERNATIONAL SEARCH REPORT**

International application No.
**PCT/JP2022/006890**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**
**Information on patent family members**

International application No.
**PCT/JP2022/006890**

| Patent document cited in search report | Publication date (day/month/year) | Patent family member(s) | Publication date (day/month/year) |
|---|---|---|---|
| WO 2010/104025 A1 | 16 September 2010 | US 2011/0318764 A1<br>claims, paragraphs [0045]-[0047]<br>EP 2407784 A1<br>CA 2753881 A1<br>AU 2010222123 A1<br>KR 10-2011-0134907 A<br>CN 102348982 A<br>RU 2011140807 A | |
| WO 2009/043015 A1 | 02 April 2009 | US 2010/0209962 A1 | |
| WO 2016/021704 A1 | 11 February 2016 | US 2018/0136241 A1<br>claims, paragraph [0073]<br>EP 3179250 A1<br>CN 106574923 A | |
| US 2012/0021437 A1 | 26 January 2012 | WO 2011/159735 A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016021704 A
- US 20120021437


### Non-patent literature cited in the description


- *J Biol Chem.,* 2009, vol. 284 (33), 22344-52
- **MAEDA S ; NAKAMURA T ; HARADA H ; TACHIBANA Y ; ARITAKE K ; SHIMOSAWA T et al.** Prostaglandin D2 metabolite in urine is an index of food allergy. *Sci Rep.,* 17 December 2017, vol. 7 (1), 17687
- **INAGAKI S ; MAEDA S ; NARITA M ; NAKAMURA T ; SHIMOSAWA T ; MURATA T et al.** Urinary PGDM, a prostaglandin D2 metabolite, is a novel biomarker for objectively detecting allergic reactions of food allergy. *J Allergy Clin Immunol.,* 10 July 2018, vol. 142 (5), 1634-6
- **TAKESHITA E ; KOMAKI H ; TACHIMORI H ; MIYOSHI K ; YAMAMIYA I ; SHIMIZU-MOTOHASHI Y et al.** Urinary prostaglandin metabolites as Duchenne muscular dystrophy progression markers. *Brain Dev.,* 15 July 2018, vol. 40 (10), 918-52
- **HIGASHI et al.** Urinary tetranor-PGDM concentrations in aspirin-intolerant asthma and anaphylaxis. *J Allergy Clin Immunol.,* 22 October 2011, vol. 129 (2), 557-9
- **SONG WL ; WANG M ; RICCIOTTI E ; FRIES S ; YU Y ; GROSSER T et al.** Tetranor PGDM, an abundant urinary metabolite reflects biosynthesis of prostaglandin D2 in mice and humans. *J Biol Chem.,* 13 November 2007, vol. 283 (2), 1179-88
- **INAGAKI S ; MAEDA S ; NARITA M ; NAKAMURA T ; SHIMOSAWA T ; MURATA T et al.** Urinary PGDM, a prostaglandin D2 metabolite, is a novel biomarker for objectively detecting allergic reactions of food allergy. *J Allergy Clin Immunol,* 10 July 2018, vol. 142 (5), 1634-6
- **TAKESHITA E ; KOMAKI H ; TACHIMORI H ; MIYOSHI K ; YAMAMIYA I ; SHIMIZU-MOTOHASHI Y et al.** Urinary prostaglandin metabolites as Duchenne muscular dystrophy progression markers. *Brain Dev,* 15 July 2018, vol. 40 (10), 918-25
- **HIGASHI N ; MITA H ; YAMAGUCHI H ; FUKUTOMI Y ; AKIYAMA K ; TANIGUCHI M.** Urinary tetranor-PGDM concentrations in aspirin-intolerant asthma and anaphylaxis. *J Allergy Clin Immunol,* 25 October 2011, vol. 129 (2), 557-9
- **HARLOW E ; LANE D.** Antibodies: a laboratory manual. Cold Spring Harbor Laboratory, 1988, vol. xiii, 726
- **QUEFFELEC AL ; BOISDE F ; LARUE JP ; HAELTERS JP ; CORBEL B ; THOUVENOT D et al.** Development of an immunoassay (ELISA) for the quantification of thiram in lettuce. *J Agric Food Chem.,* 20 April 2001, vol. 49 (4), 1675-80
- **DUDLEY RA ; EDWARDS P ; EKINS RP ; FINNEY DJ ; MCKENZIE IG ; RAAB GM et al.** Guidelines for immunoassay data processing. *Clin Chem.,* 01 August 1985, vol. 31 (8), 1264-71
- **HAYASHI Y ; MATSUDA R ; MAITANI T ; IMAI K ; NISHIMURA W ; ITO K et al.** Precision, limit of detection and range of quantitation in competitive ELISA. *Anal Chem.,* 28 February 2004, vol. 76 (5), 1295-301
- **SCHNEIDER D ; GEE S ; KREISSIG S ; HARRIS A ; KRAMER P ; MARCO M et al.** New frontiers in agrochemical immunoassay. AOAC International, 1995, 103-21
- **SMITH-PALMER T.** Separation methods applicable to urinary creatine and creatinine. *J Chromatogr B Analyt Technol Biomed Life Sci.,* 27 November 2002, vol. 781 (1-2), 93-106
- **GRANSTROM E ; SAMUELSSON B.** On the metabolism of prostaglandin F 2 in female subjects. II. Structures of six metabolites. *J Biol Chem.,* 25 December 1971, vol. 246 (24), 7470-85
- **ZHANG Y ; ZHANG G ; CLARKE PA ; HUANG JT ; TAKAHASHI E ; MUIRHEAD D et al.** Simultaneous and high-throughput quantitation of urinary tetranor PGDM and tetranor PGEM by online SPE-LC-MS/MS as inflammatory biomarkers. *J Mass Spectrom,* 28 June 2011, vol. 46 (7), 705-11